# FASCICULE DE BREVET EUROPEEN

(11) **EP 0 510 079 B1**
(45) Date de publication et mention de la délivrance du brevet: **19.05.1999**
(21) Numéro de dépôt: 91903010.6
(22) Date de dépôt: 08.01.1991
(51) Int. Cl.: C07K 14/705, C12N 15/12, A61K 38/16, C07K 16/28, C12P 21/02, C12N 15/11, G01N 33/577, A61K 39/395

(54) **PROTEINES PRODUITES PAR LES LYMPHOCYTES HUMAINS, SEQUENCE D'ADN CODANT POUR CES PROTEINES ET APPLICATIONS PHARMACEUTIQUES ET BIOLOGIQUES**
DURCH MENSCHLICHE LYMPHOZYTEN PRODUZIERTE PROTEINE, DIE FÜR DIESE PROTEINE KODIERENDE DNS UND PHARMAZEUTISCHE UND BIOLOGISCHE VERWENDUNGEN
PROTEINS PRODUCED BY HUMAN LYMPHOCYTES, DNA SEQUENCE CODING THESE PROTEINS, AND PHARMACEUTICAL AND BIOLOGICAL USES THEREOF

(30) Priorité: 08.01.1990 FR 9000126
(43) Date de publication de la demande: 28.10.1992
(73) Titulaire: INSTITUT NATIONAL DE LA SANTE ET DE LA RECHERCHE MEDICALE (INSERM), 75654 Paris Cédex 13 (FR); INSTITUT GUSTAVE ROUSSY, F-94805 Villejuif Cédex (FR)
(72) Inventeur: HERCEND, Thierry, F-94700 Maisons-Alfort (FR); TRIEBEL, Frédéric, F-92200 Neuilly (FR)
(74) Mandataire: Le Guen, Gérard
(86) Numéro de dépôt international: FR9100009
(87) Numéro de publication internationale: WO9110682

(56) Documents cités:
- EP-A- 320 806
- EP-A- 329 363
- The EMBO Journal, vol. 6, no. 12, 1987, IRL Press Ltd, (Oxford, GB), D.E. Staunton et al.: "Molecular cloning of the lymphocyte activation marker blast-1", pages 3695-3701
- J. Exp. Med., vol. 165, mars 1987, The Rockefeller University Press, J. Jongstra et al.: "The isolation and sequence of a novel gene from a human functional T cell line", pages 601-614
- Chemical Abstracts, vol. 102, no. 25, 24 juin 1985, (Columbus, Ohio, Us), N.T. Chang et al.: "A cDNA clone encoding a product of activated human T lymphocytes", voir page 181
- The Journal of Experimental Medicine, vol. 171, Mai 1990, The Rockefeller University Press, F. Triebel et al.: "LAG-3, a novel lymphocyte activation gene closely related to CD4", pages 1393-1405

## Description

La présente invention concerne des protéines produites par des lymphocytes humains et, en particulier, une protéine exprimée à la surface de ceux-ci, des séquences d'ADN codant pour ces protéines et les applications pharmaceutiques et biologiques de ces protéines.

Un certain nombre de structures protéiques de la surface cellulaire "appartiennent" à la "super-famille" des immunoglobulines (IgSF). Cette famille de molécules inclut les protéines comprenant au moins un domaine avec une région de repliement caractéristique appelée repliement Ig. Plusieurs de ces molécules possèdent des fonctions essentielles dans les réponses immunologiques.

En plus d'assurer une reconnaissance spécifique de l'antigène comme le font par exemple les immunoglobulines ou les récepteurs T, elles peuvent fonctionner comme des ligands monomorphiques essentiels dans les interactions cellule-cellule (par exemple ICAM, CD4, CD8), des récepteurs pour des virus (par exemple CD4, ICAM) ou des récepteurs pour les lymphokines (par exemple IL1-R, IL6-R).

La séquence du récepteur CD4 a été décrite par P.J. Maddon et al. (Cell, 42, 93, 1985).

La découverte et la caractérisation des protéines membranaires exprimées sur les lymphocytes ont été facilitées par la mise au point des techniques de génie génétique. Cette méthodologie permet par diverses techniques expérimentales de caractériser les gènes codant pour des protéines et donc à travers la séquence nucléotidique du gène de connaître la séquence peptidique. D'autres applications de ces techniques de génie génétique fondées sur les mêmes principes expérimentaux permettent grâce à des systèmes d'expression procaryotes ou eucaryotes de produire en quantités virtuellement infinies les protéines dont le gène a été découvert.

Les inventeurs ont cherché à découvrir de nouveaux gènes codant pour des protéines membranaires non décrites à ce jour.

Le développement des travaux des inventeurs a permis d'isoler à partir de lymphocytes cytotoxiques naturels un nouvel ADNc complémentaire (ADNc) désigné FDC. Cet ADNc code pour une protéine appelée LAG-3 (pour Lymphocyte Activation Gene-3) qui présente une séquence signal dont on pense qu'elle s'élimine dans la protéine mature.

La présente invention a, en conséquence, pour objet une séquence d'ADN comprenant la séquence nucléotidique, désignée FDC, correspondant à la séquence d'ADNc représentée sur la séquence SEQ ID N° 1.

La traduction commence au nucléotide 231 et se termine au nucléotide 1724 inclus.

La présente invention a également pour objet la protéine codée par FDC, à savoir la protéine LAG-3 représentée sur la séquence ID N° 1 (séquence protéique -28 à 470).

Les 28 premiers amino-acides devraient constituer une séquence signal qui est éliminée dans la protéine mature.

La présente invention a donc plus particulièrement pour objet la protéine correspondant à la séquence protéique 1 à 470 de SEQ ID N° 1.

La protéine mature constitue une protéine membranaire de type I de 470 amino-acides, dont la masse moléculaire théorique déduite de la structure protéique est de 51295 Daltons et de point isoélectrique égal à 10,9. Elle comporte une région extracellulaire incluant environ 420 amino-acides et, une région cytoplasmique incluant environ 24 amino-acides reliées par un peptide transmembranaire incluant environ 26 amino-acides. La partie extracellulaire de la protéine LAG-3 correspond aux amino-acides 1 à 420 de la protéine LAG-3 décrite plus haut.

La comparaison de la séquence du gène LAG-3 représentée par l'ADNc FDC ci-dessus ainsi que de l'organisation exon/intron du gène LAG-3 avec celles d'autres molécules de type Ig/SF a révélé une relation étroite de la protéine LAG-3 avec la protéine CD4.

Il est connu que les gènes des cellules eucaryotes présentent un phénomène de polytypie. Par ce phénomène, certains des amino-acides de la protéine codée sont parfois remplacés sans modification de l'activité. La présente invention englobe les protéines provenant de ce phénomène.

La présente invention a donc plus généralement pour objet une protéine ayant la séquence peptidique correspondant à la séquence SEQ ID N° 2 dans laquelle Xaa est choisi parmi H, un résidu méthionine ou la séquence -28 à -1 de la séquence protéique représentée sur la SEQ N° 1 et les séquences qui en diffèrent par un ou plusieurs amino-acides et qui se lient aux anticorps monoclonaux spécifiques d'une protéine représentée par la séquence SEQ ID No. 1.

Les inventeurs ont en outre trouvé une séquence d'ADN qui est une région promotrice d'un gène codant pour une protéine selon l'invention. Cette séquence est celle représentée sur la sequence N° 4.

La présente invention a en conséquence également pour objet cette séquence d'ADN.

La présente invention a également pour objet une séquence d'ADN comprenant la séquence d'ADN promotrice telle que définie ci-dessus et une séquence d'ADN codant pour une protéine selon la présente invention.

Dans la présente invention, les inventeurs ont tout d'abord obtenu un ADN complémentaire FDC par les opérations suivantes :
- culture de cellules lymphocytaires dites cellules cytotoxiques naturelles
- isolement à partir de ces lymphocytes de l'ARN messager lié aux membranes du réticulum endoplasmique intra-cellulaire
- obtention de l'ADN complémentaire simple brin à partir de l'ARN messager, puis de l'ADN complémentaire double brin
- insertion dans un vecteur tel que le bactériophage lambda GT10
- préparation d'une sonde d'ADN simple brin à partir de l'ARN messager des cellules et purification par une technique d'hybridation-soustraction de façon à sélectionner les copies des ARN présentes dans les cellules lymphocytaires cytotoxiques naturelles et absentes dans d'autres cellules hématopoïétiques transformées.
- sélection des ADN complémentaires insérés dans le vecteur qui réagissent avec la sonde
- transfert de l'ADN sélectionné dans un vecteur plasmidique pour l'amplifier, le purifier et en réaliser le séquençage.

La séquence protéique selon l'invention a été obtenue par :
- traduction de la séquence nucléotidique de l'ADNc FDC.

L'existence de cette protéine à l'état naturel sur les cellules T a été démontrée par :
- préparation de sérums dirigés contre un peptide synthétique représentant une région probablement exposée vers l'extérieur du produit de traduction de l'ADNc FDC, ayant une structure protéique en forme de boucle,
- immunoprécipitation de la protéine LAG-3 par les hétéro-anticorps anti-peptide.

Les protéines selon l'invention peuvent, en outre, être obtenues par d'autres méthodes de purification de protéines membranaires ou synthèse peptidique classique ou encore par application de techniques de génie génétique comprenant l'insertion d'une séquence d'ADN codant pour une protéine selon l'invention dans un vecteur d'expression tel qu'un plasmide et la transformation de cellules avec ce vecteur d'expression et culture de ces cellules.

La présente invention a donc également pour objet des plasmides et des vecteurs d'expression comprenant une séquence d'ADN codant pour une protéine selon l'invention ainsi que des hotes transformés avec ce vecteur.

La présente invention a également pour objet une composition thérapeutique comprenant à titre de principe actif une protéine selon l'invention ou une partie de cette protéine, en particulier la partie soluble correspondant à la région extracellulaire de la protéine s'étendant de l'amino-acide 1 à l'amino-acide 420 de la séquence protéique précédemment décrite ou une partie de cette région extracellulaire et notamment tout ou partie d'au moins l'une des quatre domaines extracellulaires de type immunoglobuline de la protéine LAG-3 (séquences 1 à 142, 143 à 232, 233 à 342 et 343 à 413). La partie de la protéine peut être également constitué par tout ou partie de la région intracytoplasmique (séquence 450 à 470). La partie extracellulaire peut être notamment la séquence représentée sur la séquence SEQ ID N° 3.

Cette composition thérapeutique est active dans le traitement de certaines maladies impliquant le système immunitaire où la fixation du ou des ligands de la protéine LAG-3 sur-cette protéine provoque la transmission de signaux vers l'intérieur de la cellule, ou des modifications des interactions cellulaires.

Dans ce cas, la composition objet de l'invention peut agir en fixant le ou les ligands de la protéine membranaire LAG-3 empêchant de cette manière la fixation néfaste de ce ou ces ligands sur la protéine LAG-3, par un phénomène d'inhibition compétitive.

La présente invention a également pour objet des anticorps monoclonaux dirigés contre une protéine selon l'invention ou une séquence immunogène d'une telle protéine, en particulier une séquence peptidique comprenant la séquence représentée sur SEQ N°3. La présente invention a également pour objet des hybridomes produisant de tels anticorps monoclonaux.

La présente invention englobe également les fragments et les dérivés d'anticorps monoclonaux selon l'invention qui sont réactifs avec des régions définies de la protéine LAG-3. Ces fragments sont notamment les fragments F(ab')₂ qui peuvent être obtenus par clivage enzymatique des molécules d'anticorps avec la pepsine, les fragments Fab' qui peuvent être obtenus par réduction des ponts disulfure des fragments F(ab')₂ et les fragments Fab qui peuvent être obtenus par clivage enzymatique des molécules d'anticorps avec la papaïne en présence d'un agent réducteur. Ces fragments, ainsi que des fragments Fv, peuvent être également obtenus par génie génétique.

Les dérivés d'anticorps monoclonaux sont par exemple des anticorps ou des fragments de ces anticorps auxquels sont liés des marqueurs tels qu'un radioisotope. Les dérivés d'anticorps monoclonaux sont également des anticorps ou des fragments de ces anticorps auxquels sont liées des molécules thérapeutiquement actives, notamment des composés cytotoxiques.

De plus, les anticorps monoclonaux ou les fractions solubles de la protéine LAG-3 et notamment tout ou partie d'au moins l'un des quatre domaines extracellulaires de type immunoglobuline de la protéine LAG-3 (séquences 1 à 142, 143 à 232, 233 à 342 et 343 à 413) ou la région intracytoplasmique (séquences 450 à 470) de cette protéine peuvent être utilisées dans le traitement des affections humaines dues à l'infection par les virus du type VIH.

Ces mêmes produits peuvent être utilisés dans le traitement des maladies humaines où un mécanisme pathophysiologique fait intervenir les interactions d'adhésion intercellulaire entre un ligand et LAG-3 (en particulier avec le premier et/ou le deuxième domaine externe de LAG-3) comme, par exemple, les maladies auto-immunes.

Ils peuvent également être utilisés dans le traitement des maladies humaines causées par des virus se liant de façon spécifique à la molécule LAG-3 et notamment au premier domaine externe NH₂-terminal.

La présente invention a également pour objet un procédé de dosage ou d'identification des protéines selon l'invention qui comprend l'utilisation d'anticorps monoclonaux selon l'invention.

A cet effet, on peut utiliser dans le cas où une partie de la protéine LAG-3 est soluble à l'état naturel une méthode radio-immunologique de type RIA ou de type IRMA (technique de type sandwich utilisant un antigène froid et la compétition entre un anticorps froid et un anticorps marqué) ou une méthode immunoenzymatique de type ELISA ou de type IEMA (technique de type sandwich).

Pour identifier la protéine LAG-3 fixée à la membrane, on peut utiliser des méthodes telles que l'immunofluorescence directe (par anticorps anti-LAG-3 marqués par une substance fluorescente) ou indirecte (en utilisant une immunoglobuline marquée anti-Ig de souris dans la cas où les anticorps anti-LAG-3 ont été produits dans cette espèce).

Les anticorps monoclonaux dirigés contre les protéines selon l'invention ou des fractions de celles-ci peuvent être préparés selon une technique classique. A cet effet les fractions de protéines peuvent être couplées si nécessaire à un agent immunogène, tel que l'anatoxine tétanique par un agent de couplage tel que le glutaraldéhyde.

On décrira ci-après plus en détail l'obtention de l'ADNc FDC et du gène LAG-3 codant pour la protéine en se reportant aux figures annexées sur lesquelles :
- la figure 1 représente la carte de restriction de l'ADNc FDC et les clones d'ADNc qui ont permis d'obtenir la séquence du clone FDC;
- la figure 2 représente la carte de restriction et la répartition exons-introns du gène LAG-3;
- la figure 3 est une représentation schématique de la protéine LAG-3;
- la figure 4 représente un modèle du domaine 1 de la protéine LAG-3;
- la figure 5 représente l'alignement des domaines 1 et 2 avec les domaines 3 et 4 de la protéine LAG-3;
- la figure 6 représente l'alignement des séquences peptidiques de LAG-3 et de la protéine CD4 de rat;
- la Fig. 7 est le résultat d'une immunoprécipitation de protéines membranaires de PHA-blastes;
- la Fig. 8 est un schéma de la préparation d'un vecteur de transfert (système du baculovirus) ;
- la Fig. 9 est le résultat de la détection d'un hétéroantisérum de LAG-3C dans le système du baculovirus en immunoflurorescence;
- la Fig. 10 montre le réactivité en immunofluorescence d'un hétéroantisérum sur PHA-blastes et PBL;
- la Fig. 11 est le résultat de la détection par un hétéroantisérum de LAG-3S dans le système du baculovirus en western blot.

### I - Culture et préparation de l'ARNm lié aux membranes du réticulum endoplasmique

L'isolement et les caractéristiques du clone foetal, F55IIIE5 (phénotype CD3⁻ CD2⁺) ont été décrites préalablement par Nowill et al (1).

La culture de masse a été effectuée en présence d'interleukine-2 recombinante, de surnageant de milieu conditionné lymphocytaire, sur une sous-couche nourricière de cellules sanguines mononuclées irradiées allogéniques et d'une lignée cellulaire transformée par le virus EBV (appelée LAZ 388) dans des plaques à fond en V à 96 trous. Les cellules ont été mises à 3000 cellules par puits au jour 0. Le recueil de 200 plaques à 3 x 10⁶ cellules par ml au jour 12 a permis de recueillir 6 x 10⁹ cellules.

La préparation des ARN cytoplasmiques, des ARN liés aux membranes du réticulum endoplasmique et des ARNm ont été faites en apportant certaines variantes aux méthodes décrites par Maniatis (2) Mechler (3) et Aviv (4). Ainsi 4 x 10⁹ cellules de F55IIIE5 ont été déposées sur des gradients de saccharose après choc hypotonique et broyage mécanique selon la méthode décrite par Mechler. Les ARN cytoplasmiques portés par les ribosomes liés aux membranes du réticulum endoplasmique ont été purifiés entre des gradients de saccharose. Ceci permet de travailler par la suite avec des ARNm qui ont une séquence signal et qui par conséquent codent pour des protéines portées par la membrane ou secrétées dans la partie interne de l'ergastoplasme (et vers l'extérieur de la cellule). Cette méthode d'isolement de l'ARN de type dit MB (membrane-bound) permet d'emblée d'éliminer environ 90% des gènes transcrits qui codent pour des protéines intra-cellulaires ne pouvant pas être secrétées vers l'extérieur ou transportés vers la membrane et par conséquent sans intérêt dans le cadre de l'invention. En plus de l'isolement du ARNm MB-F55IIIE5 qui sert de substrat à d'une part la construction de la banque et d'autre part la fabrication de la sonde, les méthodes de purification décrites par Aviv (4), Maniatis (2) et Triebel (5) ont permis d'isoler des ARN de divers clones et lignées cellulaires qui sont utilisés et des ARNm des cellules Jurkat, U937, Laz388 et K562 (envi-ron 10⁹ cellules de chacune des lignées) qui sont utilisés pour soustraire la sonde.

Ces méthodes comprennent :
A - Préparation de l'ARN cytoplasmique.
   A une ampoule de 20 à 30.10⁶ cellules en culot sec, on ajoute 1 ml de tampon de lyse (Tris HCl 50mM, EDTA 62,5 mM, Triton X-100 0,4%, LiCl 2,5 M). Après dissolution douce du culot, on transfère le tam-pon de lyse dans les tubes Eppendorf froids, contenant 50 µl de NP40 10%.
   Après 5 mn sur glace, on centrifuge 1 min à 8000 trs/min. On prélève le surnageant (ARN) et on l'ajoute dans les tubes Falcons contenant 1 ml de phénol, 1 ml de CHCl3, 1 ml de STE SDS 2% (NaCl 150 mM, Tris 10 mM, MgCl₂ 1 mM, 2% SDS). On centrifuge 10 mn à 5000 trs/min. On prélève la phase supérieure, on ajoute 1 ml de phénol et 1 ml de chloroforme. On centrifuge 5 mn à 5000 trs/min. On prélève la phase supérieure. On ajoute 100 µl EDTA 0,2 M, 200 µl NaAc 3M et 5 ml éthanol. On mélange et on laisse à - 20°C la nuit. On centrifuge 30 mn à 10000 trs/min. On sèche le culot. On reprend dans 400 µl de NaAc 0.3 M froid. On ajoute 1 ml d'éthanol dans le tube Falcon. On transfère l'éthanol dans l'Eppendorf, on mélange, on laisse 1h à - 20°C. On centrifuge 10 min à 13 K, aspire l'alcool et sèche. On ajoute 30 µl d'eau. On centrifuge, on congèle immédiatement à - 80 °C. On contrôle la dégradation et la quantité en mettant 1 µl sur gel dénaturant (1% agarose dans tampon TBE (Tris, Base, EDTA) pH 8,5 autoclavé (BET 1 µg/ml).
B - Préparation de l'ARN messager lié aux membranes du réticulum endoplasmique
   On reprend les cellules à 10⁸ cellules/ml dans du tampon hypotonique RSB (KCl 10mM, MgCl₂ 1,5 mM, Tris-HCl 10mM pH 7,4) à la température de la glace traité préalablement avec 0,1% DEPC. On laisse 5 min sur la glace. On rompt les cellules mécaniquement avec 10 coups d'homogéniseur de Dounce (type B). On centrifuge à 1000 g pendant 2 min pour sédimenter les noyaux. Le surnageant ou "extrait cytoplasmique" est ensuite utilisé pour la séparation ribosomes libres/extraits membranaires. 0,7 ml d'extrait cytoplasmique est mélangé avec 3,2 ml de tampon 2,5 M saccharose TK (Tris-HCl 0,05 M, pH 7,4, KCl 0,15 M, MgCl₂ 0,005 M) puis ce mélange est déposé sur 2 ml de 2,5 M saccharose TK. Puis on ajoute 8 ml de 2,05 M saccharose TK et ensuite 4 ml de saccharose 1,3 M TK. On fait tourner à 4°C pendant 5 h dans un rotor pendulaire type Spinco SW28 à 25000 trs/min. On pique -avec une aiguille la bande comprise entre le gradient de 2,05 M et celui de 1,3 M de saccharose. On ajoute un volume égal de TE 10 : 1 (Tris HCl 10mM, EDTA 1mM). On fait une extraction avec du phénol puis un mélange phénolchloroforme. On précipite avec 1/10 de NaAc 3 M et 2,5 vol. d'éthanol.
   Pour la sélection de l'ARN poly (A)⁺ on utilise une colonne d'oligo (dT)-cellulose comprenant 1,2 ml de gel équilibrée avec le tampon de charge : Tris-HCl 20mM (pH 7,6), Nacl 0,5 M, EDTA 1mM complété avec SDS. On lave avec H₂O, une solution NaOH 0,1 M et EDTA 5mM et de l'eau. On lave avec 5 volumes de tampon de charge. On dissout l'ARN dans de l'eau et on chauffe à 65°C 5 min. On ajoute deux fois un volume identique de tampon de charge. On attend que la température s'équilibre. On récolte l'effluent. On chauffe à 65°C et on recommence. On lave la colonne avec 5 à 10 volumes de tampon de charge puis 4 volumes de tampon de charge NaCl 0,1 M. On élue le poly(A)⁺ avec 2-3 volumes de Tris-HCl (pH 7,5) 10mM, EDTA 1mM, SDS 0,05%.
   On ajoute de l'acétate de sodium 3 M (pH 5,2) au 1/10. On précipite avec 2,2 vol. d'éthanol.

### II - Obtention de la banque d'expression

La préparation de l'ADN complémentaire simple brin à partir de l'ARN messager lié aux membranes du réticulum endoplasmique cellulaire de la cellule F55IIIE5 puis de l'ADN complémentaire double brin in vitro ont été réalisés selon les techniques décrites par Gubler et al (6).

Après protection des sites internes EcoRI par la méthylase EcoRI et sélection des tailles sur gel d'agarose à basse température permettant de sélectionner des fragments dont la taille est supérieure à 500 pb, les ADNc double-brin ont été clonés dans le site EcoRI du phage Lambda GT10 à l'aide du linker EcoRI.

L'empaquetage in vitro des phages Lambda GT10 recombinants a été réalisé en utilisant un kit de clonage commercial (Amersham Corp. Arlington Heights, IL).

Après culture sur E. coli C 600 Hfl⁺, on obtient 6 x 10⁴ phages recombinants.

### III - Préparation de la sonde d'ADN complémentaire

La préparation de la sonde d'ADN complémentaire simple brin est effectuée par soustraction par deux cycles d'hybridation sur un excès d'ARN messager des cellules dites "à éliminer" (Jurkat, Laz 388, U937, K562) suivi de passages sur colonnes d'hydroxyapatite, ce qui permet de se séparer du complexe double brin ADNc-ARNm. Après 2 cyles d'hybridation et 2 passages sur colonne il reste environ 6-7% de la radioactivité, c'est-à-dire qu'environ 7% du matériel dit MB ("membrane-bound") F55IIIE5 n'existe pas dans les cellules Jurkat, K562,U937 et Laz 388. C'est ce matériel qui sert de sonde pour aller rechercher dans la banque MB-F55IIIE5 les ADNc correspondants. Cette technique utilise les conditions d'hybridation -soustraction décrites par Davis et al (7).

Préparation de la sonde soustraite /MB-FSSIIIES -ArNm de Jurkat, K562,Laz 388,U937/

A partir de 5 µg de ARNm MB-F55IIIE5 on prépare une sonde d'ADNc simple brin marquée au ³² P-dCTP (activité spécifique : 800 Ci/mmol⁻¹) dans un volume de 50 µl.

Après incubation de 2 h à 42°C avec l'enzyme transcriptase réverse, on ajoute 5 µl EDTA 0,2 M puis 50 µl NaOH 0,2 N. On incube à 65°C, 1 h. On ajoute 60 µl HCl 1N et 30 µl Tris-HCl (pH 8) 2 M. On ajoute 1/10^{e} vol. NaAc 3M. On ajoute 7 µl d'ARNm de chacune des 4 lignées tumorales pour précipiter la sonde, puis on ajoute 2,5 vol. d'éthanol.

On laisse 1 h à -20°C. On centrifuge, on lave à l'éthanol 70% et on sèche. On reprend dans 7,5 µl d'H₂O, on ajoute 7,5 µl NaH₂PO₄ 0,5, M pH 7, 1mM EDTA, 0,25% SDS. On incube à l'étuve 68°C pendant 20 heures.

On dilue le contenu dans 1 ml NaH₂PO₄ 0,12 M, 0,1% SDS. On charge sur une colonne d'hydroxyapatite équilibrée à 60°C dans le même tampon. L'effluent (matériel simple brin) est concentré par du sec-butanol et passé sur une colonne G-50 pour enlever le tam-pon phosphate. On ajoute de nouveau 7 µg de ARNm de chacune des lignées et on recommence l'hybridation et le passage sur colonne. Après ces 2 passages, on récupère 7% de la quantité de la radioactivité du départ.

### IV - Isolement et caractérisation des clones d'ADNc

La banque d'ADNc obtenue précédemment (2 x 10⁴ phages recombinants) est ensemencée sur E. coli C600/Hfl. Le criblage est effectué selon les techniques habituelles en utilisant des filtres de nylon comme décrit par Huynh (8).

L'hybridation avec la sonde obtenue précédemment est effectuée à 42°C avec préhybridation avec une solution d'hybridation de type Southern et addition de la sonde soustraite MB - F55IIIE5 simple brin à 5.10⁶ cpm/ml.

Après deux cycles d'hybridation-soustraction, on identifie 120 phages Lambda GT10 positifs sur les 2.10⁴ recombinants.

La mise en culture des phages positifs, la purification des ADN correspondants, la purification des ADN complémentaires sous forme de fragments par excision à partir d'une électrophorèse sur gel d'agarose à point de gélification bas ont été réalisés selon les méthodes décrites par Maniatis (2) et Huynh (8).

La ligation des ADNc les plus longs dans le vecteur plasmidique pBS digéré par l'endonucléase EcoRI et traité par la phosphatase alkaline d'intestin de veau, la transformation de bactéries JM 109 compétentes et le criblage des recombinants par un double système de sélection (ampicilline + X-gal/IPTG) ont été effectués selon des méthodes de génie génétique classiquement utilisées.

La purification et la préparation en quantité importante des ADN complémentaires recombinants clonés dans pBS ont été réalisés en utilisant la méthode de purification par gradient sur chlorure de césium décrite par Maniatis (2).

On a isolé un clone d'ADNc que l'on a nommé FD47 qui comportait 400pb et s'hybridait avec la sonde obtenue par hybridation-soustraction. Ce clone a été retenu d'une part parce que dans les techniques de "Northern blot" il s'hybridait avec un transcript de 2 kb constamment retrouvé dans les cellules F55IIIE5 et non retrouvé dans les cellules Jurkat, Laz 388, K 562 et U937 et, d'autre part, parce qu'il ne présentait pas d'homologie avec l'une quelconque des séquences connues de la banque de données intitulée "Genbank". Le clone FD47 portait une région nucléotidique pouvant coder pour une région transmembranaire hydrophobe.

### V - Isolement et structure d'un ADN complémentaire complet

Parmi les 120 phages Lambda GT10 positifs obtenus après soustraction-hybridation, on n'a pas observé d'autre phage présentant une hybridation croisée avec FD47.

Afin d'établir la séquence d'ADNc dite FDC, on établit trois nouvelles banques d'ADNc à partir soit d'amorces d'oligo-dT, soit d'un hexamère de séquence aléatoire ou soit d'une amorce spécifique comprenant les nucléotides 704 à 688 de FDC. D'autre part, on réalise une sonde d'ARN simple brin marquée au ³²P à partir de FD47 par transcription in vitro à partir du plasmide pBS à l'aide de la polymérase T7 en présence d'UTP-³²P (800 Ci.mmole⁻¹) selon la méthode décrite par Triebel (5). Les trois banques sont réa-lisées à partir d'ARN messager provenant de clones CD3⁺ portant les chaînes γ et δ du récepteur T et qui transcrivent un message LAG-3 en quantité importante lorsque leur ARN est testé avec la sonde FD 47.

La sonde FD47 est utilisée pour cribler la première banque d'ADNc, ce qui permet d'obtenir le clone FD19.

De la même manière que précédemment, on marque un fragment génomique Bam HI - Hind III de 0,3 kb comprenant la partie la plus 5' de l'exon IV en utilisant comme amorce un hexamère aléatoire et on l'utilise pour cribler la seconde banque ce qui permet d'obtenir les clones FD 61 et FD 101, et la troisième banque pour obtenir un cDNA contenant de manière presque complète l'extrémité 5', appelé FD191.

Les séquences des clones FD47 et FD19 ont été déterminées directement dans le vecteur pBS par la méthode de Sanger (9) à l'aide d'une amorce M13 universelle ou une amorce M13 inversée et la polymérase T17 modifiée.

Les séquences de FD61, FD101 et FD191 ont été déterminées à partir d'ADN simple brin après clonage dans le vecteur M13mp18.

Après différents cycles d'hybridation de proche en proche ("DNA walking") en utilisant les 3 banques d'ADNc obtenues à l'aide d'amorces différentes, on a isolé ainsi des clones d'ADNc dont les séquences se chevauchent et qui couvrent un total de 1,8 kb.

L'ensemble des séquences nucléotidiques totales de ces ADNc appelée "séquence FDC" comportant 1871 pb indique que l'ARN messager du gène LAG-3 a un cadre de lecture long et ouvert et code pour une protéine de 498 amino-acides dont on obtient la séquence peptidique par déduction à partir de la séquence nucléotidique de l'ADNc.

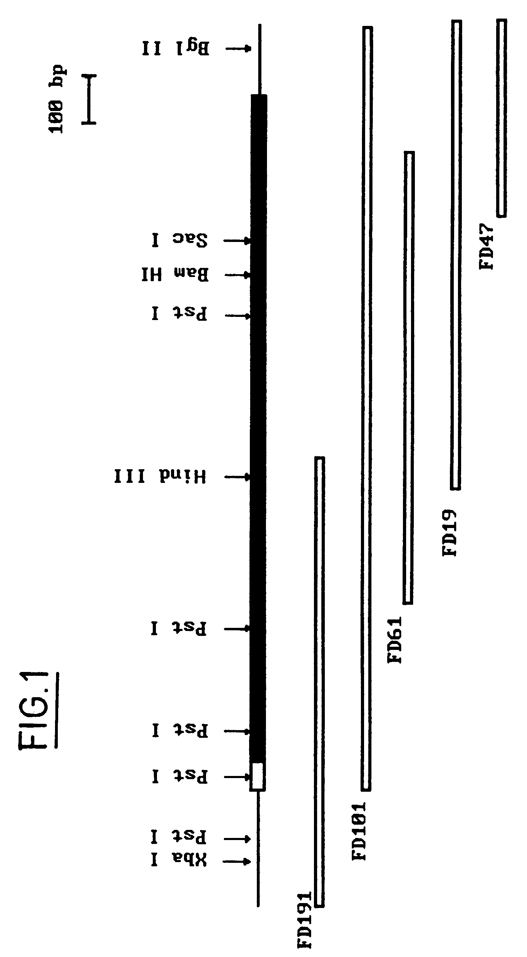

L'ADNc FDC, lui-même, a été obtenu par ligation des 2 fragments complémentaires EcoRI-Hind III, l'un couvrant la partie 5' du clone FD191, l'autre couvrant la partie 3' du clone FD19, obtenant ainsi un clone couvrant toute la séquence connue, comme illustré par la Fig. 1.

### VI - Isolement et structure du gène LAG-3

### A/ Clonage moléculaire du gène LAG-3

On isole des clones d'ADN génomique à partir de la banque LY67 obtenue à partir d'ADN d'une lignée de cellules B humaines transformées par EBV, digéré partiellement par Mbo-I et inséré dans le phage Lambda 2001 comme décrit par Dariavach (10). On marque le segment d'insertion FD47 par la méthode des amorces hexaméres aléatoires décrites par Feinberg (11) et on l'utilise pour cribler 2 x 10⁵ plaques de la banque d'ADN de génome humain. On isole neuf plaques positives (GD1 à GD9) et on caractérise des ADN de phages par des cartes de restriction à l'aide de la sonde FD19 contenant la moitié de la région codant pour la protéine et la région 3' non traduite.

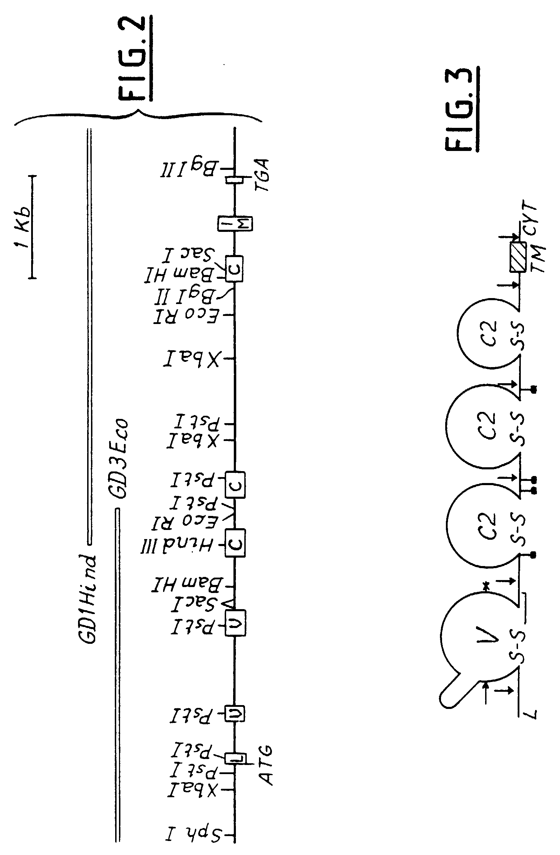

Deux fragments d'ADN se recouvrant correspondant à 16,4 kb (EcoRI) et 11,5 kb (Hind III) sont obtenus et sous-clonés dans le plasmide pUN121 pour produire les clones GD3Eco et GD1 Hind, comme représenté sur la Fig. 2.

On construit des cartes de restrictions détaillées de ces sous-clones et on les compare avec la carte de restriction de la séquence FDC de la fig. 1.

De nombreux fragments sont obtenus sur gel d'agarose à point de gélification bas et sont sous-clonés dans les bactériophages M13mp18 ou M13mp19. Les séquences de ces fragments sont obtenues à partir d'ADN simple brin à l'aide de la technique de terminaison de chaîne di-deoxy décrite. On synthétise des oligonucléotides de 17 bases dont les séquences sont obtenues soit à partir d'ADNc de FD19, soit de la séquence de la région adjacente 5' du gène LAG-3 et on les utilise dans un but de séquençage.

### B - Structure du gène LAG-3

La Fig. 2 illustre l'organisation exon-intron du gène LAG-3 humain. La carte a été construite après digestion simple et double par des endonucléases des clones GD₂ et GD₃ obtenus à partir de Lambda 2001 et de leurs sous-clones GD₃ ECO et GD₁ Hind. Les régions non traduites sont représentées par un trait fin.

Le gène LAG-3 comprend approximativement 6,6 kb et est divisé en 8 exons dont les premiers nucléotides sont respectivement ceux en position 1, 289, 437, 742, 1012, 1288, 1531 et 1662 de la séquence d'ADN précédemment décrite.

La région dite promotrice en position 5' du gène LAG-3 dont la séquence a été décrite précédemment a été étudiée et a permis les observations suivantes :
- on ne retrouve pas de séquence TATA caractéristique en amont de la région 5' non traduite de 239 pb;
- la séquence nucléotidique comprend une séquence CCAAT inversée (ATTGG) en position -662 à partir de la séquence ATG donnant le signal de traduction.

La séquence CCAAT est connue pour être cruciale dans de nombreux promoteurs et peut fonctionner dans l'orientation inversée.
- on trouve également en position -389 à partir de la séquence ATG donnant le signal de traduction un site de liaison Sp1 contenant le groupe hexa-nucléotidique caractéristique GGGCGG.

Pour évaluer le nombre de copies du gène LAG-3 dans le génome humain, on met à digérer de l'ADN de la lignée cellulaire tumorale K562 et de l'ADN de lignées cellulaires polyclonales T et NK IL₂ dépendantes avec EcoRI, Hind III, Bam HI ou XbaI. On réalise des hybridations de type Southern Blot en utilisant la sonde FDC, (1871 pb) construite par fusion du fragment 5' ECORI/Hind III du clone FD 191 avec le fragment 3' Hind III/EcoRI du clone FD 19. On obtient avec EcoRI 3 fragments de 2, 8,2 et 10 kb, avec Hind III, 2 fragments de 5,7 et 9,5 kb, avec Bam HI, 3 fragments de 2,8, 4 et 13 kb et avec XbaI 3 fragments de 3, 4 et 6 kb.

Ces résultats indiquent qu'une seule copie du gène LAG-3 est présente dans le génone haploïde humain. De plus, l'analyse des cellules T, B et NK par la même technique montre qu'il n'y a pas dans ces cellules de réarrangement du gène LAG-3 au cours de la différenciation des lymphocytes.

### VII - Expression du gène LAG-3

On a utilisé le fragment inséré de 1004 bp du clone FD19 comme sonde pour analyser la distribution cellulaire de l'expression et de la régulation de l'expression du gène LAG-3.

Les résultats du "blotting" d'ARN montrent clairement que les procédés d'hybridation-soustraction utilisés dans le premier criblage de la sous-banque F55IIIE5 ont été réalisés avec succès en ce qui concerne l'isolement du clone FD19 de la banque d'ADNc dans la mesure où aucun transcript de LAG-3 n'est exprimé dans les lignées de cellules transformées T, B et d'origine myéloïde (en particulier Jurkat, Laz 388, K 562, U 937).

On a réalisé des essais sur d'autres lignées de cellules T transformées comprenant CEM et-MOLT-4 et on a trouvé qu'aucune n'exprimait LAG-3. Il en était de même pour les monocytes circulants périphériques.

On a par ailleurs testé un échantillonnage de lignées polyclonales ou de clones de cellules normales T et NK mises en culture. Dans ce dernier cas, on a mis en évidence de l'ARN messager de LAG-3 en tant qu'échantillon unique d'environ 2kb dans toutes les lignées étudiées : 3 lignées CD3⁻ (F55 III E5, SIIH4, SIII G5), 4 lignées CD3⁺ TCRα/β⁺ (CD4⁺ : SIF8 et F55IIIG5 et une lignée CD3⁺ TCRT/δ⁺ (le clone TCRδ1⁺ TiτA⁺ BK).

Cependant, on n'a pas retrouvé d'ARN messager dans des cellules T purifiées fraîches, ni dans les macrophages périphériques, ni dans les lymphocytes au repos, dans les limites de détection généralement admises pour cette technique.

L'expression du gène LAG-3 a en outre été étudiée dans les tissus nerveux d'origine neuroectodermique et aucun ARN messager n'a été retrouvé ni dans les lignées de cellules de neuroblastome en culture, ni dans le tissu cérébral frais.

Le gène LAG-3 n'est exprimé dans les cellules T et NK qu'après activation.

L'expression du gène LAG-3 est maximale 3 à 4 jours après activation des lymphocytes du sang par la phytohémaglutinine. La protéine correspond donc à ce qu'il est convenu d'appeler un antigène d'activation.

### VIII - Structure de la protéine LAG-3

Les caractéristiques de la protéine LAG-3, représentées sur les Figures 3, 4 et 6, ont été déduites d'après la structure du gène et d'après l'analyse de son produit de traduction. Elle apparaît comme une protéine membranaire de type I comportant 498 aminoacides.

Comme représenté sur la fig. 3 les domaines sont désignés par L (domaine d'initiation), V (domaine de type V d'immunoglobuline), C₂ (domaine de type C₂ d'immunoglobuline)(19), TM (transmembranaire) et CYT (cytoplasmique). La position des introns est indiquée par des flèches. Les sites de N-glycosylation, et la séquence RGD (sites de liaison cellulaire) sont également indiqués.

La protéine mature comprend 470 aminoacides avec une masse moléculaire théorique de 51295 Daltons et un point isoélectrique de 10,9 par analyse de la structure protéique. Elle comprend un peptide de tête L (28 aminoacides) codé par les exons I (19 aminoacides) et II (9 aminoacides parmi 50). La région extracellulaire est codée par les exons II (41 aminoacides parmi 50), III (101 aminoacides), IV (90 aminoacides), V (92 aminoacides) et VI (81 aminoacides), la région transmembranaire (TM) par l'exon VII (44 aminoacides) et la région cytoplasmique incluant des aminoacides fortement chargés par l'exon VIII (21 aminoacides). La région extracellulaire comporte 8 résidus cystéine et 4 sites potentiels de N-glycosylation (Asn-X-Ser,Thr).

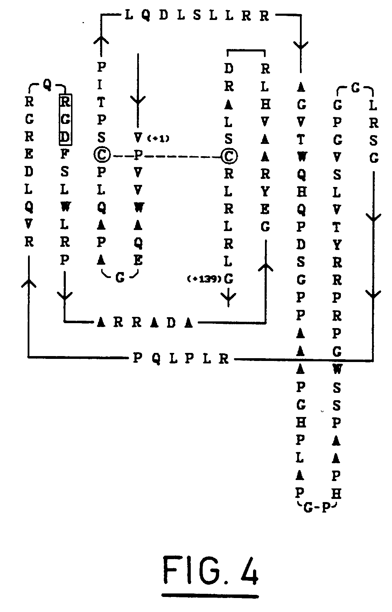

La fig. 4 représente un modèle du domaine 1 de la protéine LAG-3. La séquence du premier domaine de type Ig (aminoacides + 1 à + 139) est représentée selon le modèle utilisé par Amzel et Poljak (12). Le pont disulfure est indiqué et la séquence RGD encadrée.

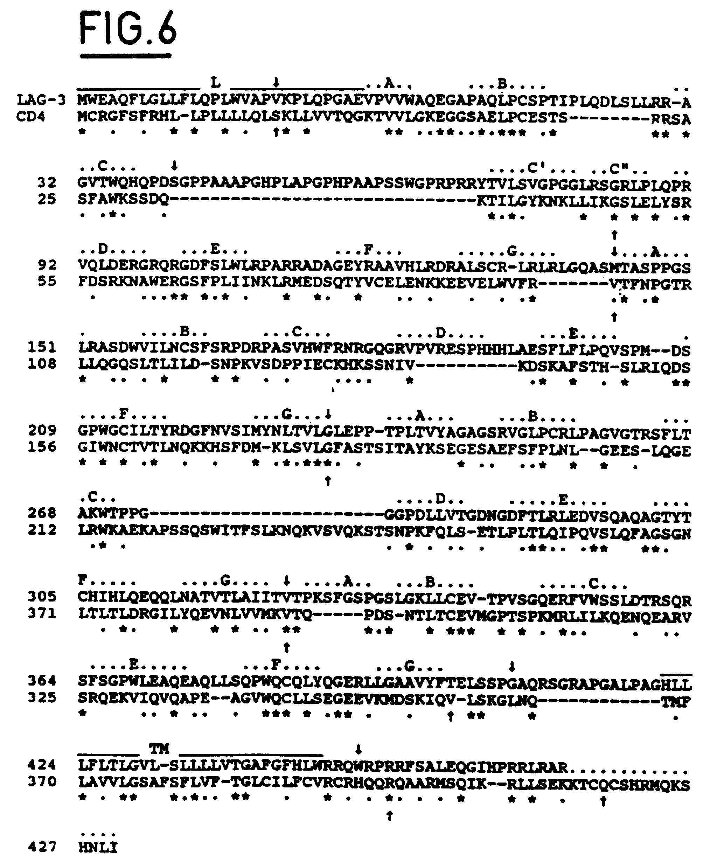

Le segment peptidique codé par les exons II et III correspond à un domaine IgSF de type V tel que décrit par Williams (13) comprenant les feuillets A, B, C, C', C", D, E, F et G représentés sur la fig. 6, possédant deux caractéristiques inhabituelles.

Premièrement, ce domaine de type V comprend une boucle additionnelle ayant approximativement 30 aminoacides codée par la première partie de l'exon III. Cette boucle représentée sur la Figure 4 joint le feuillet-β C au feuillet-β C' et contient en particulier dix résidus proline. Il semble qu'une telle insertion puisse être compatible avec un repliement de type IgSF dans la mesure où il ne provoque pas de rupture du noyau central du repliement que l'on considère comme étant constitué par les feuillets-β A, B, E et G, F, C tel que décrit par Lesk (14).

Cette boucle additionnelle a servi d'immunogène car elle est probablement exposée à l'extérieur de la mo-lécule et par conséquent exposée à une reconnaissance par des anticorps.

Les différences dans les domaines de type V et de type C apparaissent d'une façon générale au milieu du repliement de type Ig à cet endroit, c'est-à-dire au niveau du feuillet C.

De plus, dans le domaine 4 de la molécule N-CAM, il a été décrit à ce niveau l'insertion d'une structure peptidique codée par un exon supplémentaire (15) formant une mini-boucle additionnelle.

Le second point inhabituel est que la cystéine en aval du domaine 1 semble être positionnée sur le feuillet-β G plutôt que sur le feuillet-β F (résidu 121) comme cela est presque invariablement le cas. Le motif Asp-Gly-Tyr-Cys est de manière très caractéristique positionné sur le feuillet-β F et se retrouve ici, sauf qu'un résidu Ala remplace le résidu Cys (Fig. 4). Il semble possible qu'un pont disulfure puisse se former et, par exemple il est à noter qu'un pont disulfure inhabituel d'un type différent a été observé dans le domaine de type V de la chaîne α de CD8 comme décrit par Kirszbaum (16).

On trouve une séquence Arg-Gly-Asp (RGD) sur le feuillet-β E (Figure 4). Ce motif est connu pour représenter un adhésiotope potentiel comme décrit par Ruoslahti (17) mais il n'est pas établi qu'il forme le centre d'une séquence de liaison essentielle dans la mesure où, dans cette position, une telle séquence se trouverait problablement à l'intérieur du repliement de type IgSF.

Les exons IV, V et VI codent pour des domaines de type apparentés aux IgSF comme décrit par Williams (13) ayant respectivement 51, 50 et 42 amino-acides entre les deux résidus cystéine conservés. Ces trois domaines possèdent des repliements de type C et montrent des fragments de séquence caractéristiques du domaine de type C2 (13). Ils ont été comparés aux séquences du domaine de type C2 à l'aide du programme ALIGN selon la méthode décrite par Dayhoff (18) et Williams (19). Sur 57 séquences examinées, on a obtenu des scores supérieurs à 3DS, (déviations standards) 32, 41 et 11 fois pour les domaines respectifs 2, 3 et 4. Le domaine 4 appartient au domaine de type C2 tronqué dans la mesure où il ne possède pas le feuillet -β D.

Les domaines 1 et 2 de LAG-3 ont été alignés et comparés visuellement avec les domaines 3 et 4 en prenant en compte les identités et les considérations structurales.

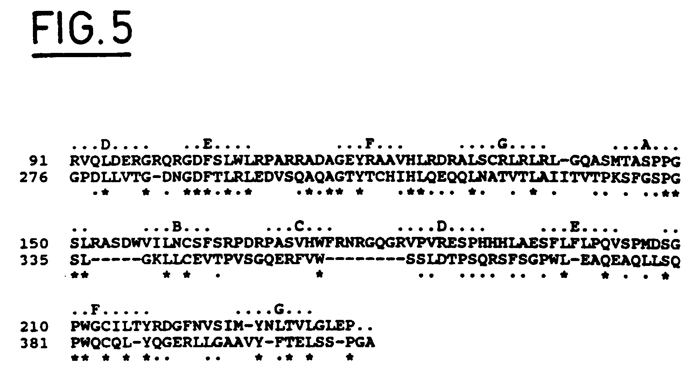

La fig. 5 représente l'homologie interne de LAG-3.

Les séquences d'aminoacides du domaine 1 (en partant de la position 91 de la Fig. 5 (et selon la numérotation de la Fig. 5) après la boucle additionnelle) et du domaine 2 ont été alignées avec les positions correspondantes dans les domaines 3 et 4. Les identités sont indiquées par des (*) et les similarités par des (.).

Dans la mesure où le domaine 1 contient une séquence formant une boucle additionnelle, l'alignement a été commencé à l'acide aminé 91 de la Figure 5 dans ce domaine et à l'amino acide 276 dans le domaine 3. Sur les 129 associations possibles entre les résidus on a observé 34 identités, 35 similarités et 9 coupures de séquences (score d'alignement supérieur à + 8,5 DS). En outre, sur le feuillet-β F des domaines 2 et 4, il existe une séquence WxC qui est tout à fait inhabituelle en cette position où on trouve habituellement la séquence Y ou FxC comme décrit par Williams (13). L'ensemble de ces résultats suggère que LAG-3 a évolué par dédoublement génique à partir d'une structure préexistante à deux domaines du type d'une chaîne Ig L.

On a également aligné les séquences de LAG-3 et de CD4 de rat, comme représenté sur la fig. 6. Les lignes en pointillés au-dessus des séquences indiquent les positions des feuillets-β dans les quatre domaines de type IgSF. La séquence de tête L et la séquence transmembranaire (TM) sont indiquées par une ligne continue au-dessus de la séquence. La position des introns est indiqué par des flèches au-dessus de la séquence (pour LAG-3) et en-dessous de la séquence (pour CD4) comme décrit par Maddon (20) pour le CD4 humain. Deux grands intervalles sont insérés correspondant à la séquence de la boucle additionnelle dans le domaine 1 de LAG-3 et pour tenir compte du fait que le domaine 3 de CD4 est du type appartenant au domaine de type V, tandis que le domaine 3 de LAG-3 est du type appartenant au domaine de type C2. Les fragments de similarités comprennent le début du domaine 1 (9 identités et 10 similarités sur 17 associations possibles), et la très inhabituelle séquence WxC dans les domaines 2 et 4 de LAG-3, qui sont également présentes en les positions correspondantes dans CD4. Ce motif de séquence n'est retrouvé en position équivalente sur aucun autre domaine de type IgSF. En tout, il y a 87 identités et 82 similarités sur 338 résidus alignés (19 coupures de séquences) lorsque on compare les régions extracellulaires de LAG-3 et de CD4 de rat. Une des caractéristiques principales de LAG-3 est par conséquent sa relation avec CD4.

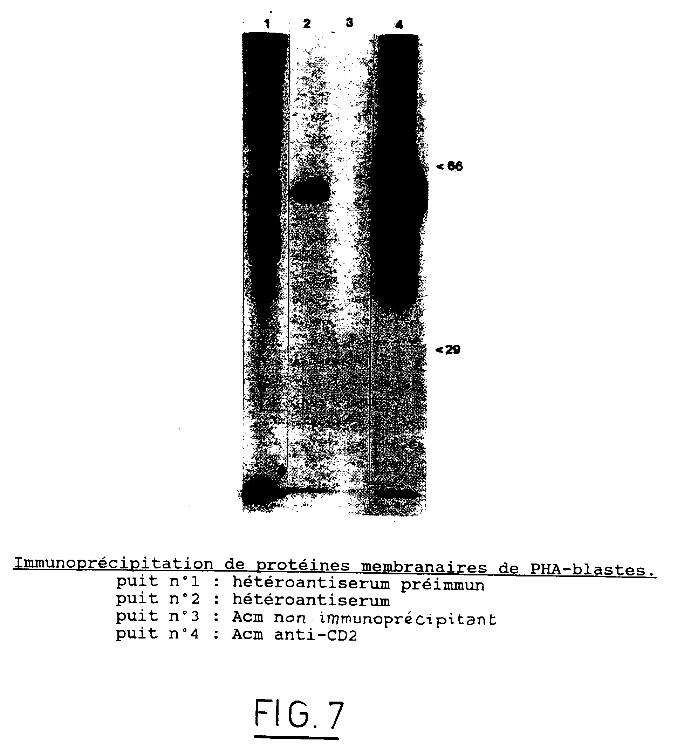

Tout comme dans la structure de LAG-3 des fragments connus ayant des homologies de séquence internes ont été retrouvés dans la molécule CD4 entre les domaines 1 et 3 aussi bien qu'entre les domaines 2 et 4. Plus généralement, l'organisation intron/exon de LAG-3 et de CD4 est très similaire : les deux gènes comprennent un intron à l'intérieur du premier domaine de type IgSF et la position des introns (indiquée par les flèches sur la Figure 7) dans LAG-3 est très proche de celle de CD4.

Il a été suggéré que CD4 avait évolué par dédoublement génique à partir d'une structure préexistante à 2 domaines de type IgSF. La présente découverte renforce cette hypothèse et les inventeurs proposent, sur la base des similarités de séquence et de l'organisation exon/intron, que CD4 et LAG-3 ont alors partagé un ancêtre commun à 4 domaines.

La protéine LAG-3 fonctionnerait donc comme le font de nombreuses autres molécules de la super famille de type Ig, comme ligand pour une protéine soluble ou pour une protéine membranaire. Les exemples connus comprennent des protéines dont l'expression est régulée positivement par activation cellulaire telle que ICAM-1, connue pour être impliquée dans les interactions cellule-cellule, ou IL1-R et IL6-R qui fonctionnent comme des récepteurs de facteurs de croissance.

Etant donné que la protéine LAG-3 est exprimée en quantité substantielle sur les lymphocytes activés (probablement plus de 5000 sites par cellule compte tenu des limites de détection des techniques d'immunofluoresence indirectes avec un anti-sérum de lapin en cytométrie de flux) et compte tenu de son homologie avec CD4, la fonction très probable de LAG-3 est une fonction d'adhésion intercellulaire. La caractérisation des couples récepteurs-ligands (par exemple ICAM-1/LFA-1 ou CD4/CMH, classe II) dans ce domaine est en cours. La molécule CD4 a été cristallisée et sa structure atomique déduite par analyse aux rayons X (Ryu (22) Wang (23)). Les sites de fixation des anticorps anti-CD4, des sites de liaison de la protéine gp120 du VIH (virus du SIDA) et les sites de fixation des molécules de classe II du complexe majeur d'histocompatibilité (CMH) ont été étudiés et il est devenu clair que le premier domaine NH₂-terminal (domaine 1) est le plus important pour l'activité fonctionnelle de CD4. Il a été montré que des molécules de CD4 solubles obtenues par délétion des parties transmembranaires et intracytoplasmiques de la molécule CD4 naturelle soit seules soit couplées à des régions constantes d'immunoglobulines (création d'une immunoadhésine CD4 (Byrn 24)) étaient capables de lier la protéine gp120 et d'empêcher la propagation de l'infection par le VIH. De même, en ce qui concerne la molécule ICAM-1, il a été montré que le premier domaine NH₂-terminal (domaine 1) contient les sites de liaison à LFA-1 et les sites de fixation des rhinovirus (Staunton (25)). Deux applications thérapeutiques qui dérivent de la connaissance de la structure de ICAM-1 ont été décrites. L'expression de ICAM-1 est fortement augmentée à la surface de l'épithélium bronchique au cours de la maladie asthmatique et dans un modèle de singe cynomegalus rendu asthmatique, il est possible de diminuer l'infiltration des bronches par des granulocytes éosinophiles et d'améliorer l'état clinique par injection intraveineuse d'anticorps anti-ICAM (Wegner (26)). En ce qui concerne l'utilisation d'une molécule recombinante rendue soluble par délétion des domaines transmembranaires et intra-cytoplasmiques, il a été montré que la molécule ICAM-1 soluble inhibe l'infection de cellules humaines par des rhinovirus en bloquant par compétition la fixation du virus sur la molécule ICAM-1 naturelle exprimée à la surface des cellules (Marlin (27)).

Il est donc possible étant donné les analogies de structure avec CD4, que LAG-3 puisse être une porte d'entrée pour un virus. En ce qui concerne le VIH ou des virus apparentés, un des sites de fixation possible comprendrait (par analogie avec CD4) dans ce cas tout ou partie de la séquence d'amino-acides suivante incluant notamment le feuillet-β C" du domaine V : Gly Leu Arg Ser Gly Arg Leu Pro Leu Gln Pro Arg Val Gln Leu Asp Glu Arg (correspondant aux aminoacides 80 à 97 de la protéine LAG-3, seq ID N° 2). Par ailleurs, le gène LAG-3 a été localisé au chromosome 12 (bande p 13.3) (Triebel (28)) proche de CD4. L'étude de cellules hybrides homme-hamster a montré que des protéines cellulaires codées par des gènes du chromosome 12 humain (gènes différents de CD4) étaient nécessaires et suffisantes pour l'expression des gènes du VIH (Hart (29)). La protéine LAG-3 pourrait donc intervenir dans le cycle reproductif du VIH, aboutissant à la production de virions infectieux, indépendamment de son éventuelle capacité à servir comme récepteur au virus VIH.

Enfin, il existe des homologies structurales entre la région intracytoplasmique de la protéine LAG-3 (notamment au niveau du peptide Arg Arg Gln Trp Arg Pro Arg Arg Phe Ser) et différentes protéines codées par le génome du VIH, comme par exemple la protéine REV (Yourno (30)) ou VIF (Ratner (31)). Ces relations d'homologies suggérent là encore que la protéine LAG-3 exprimée par les lymphocytes activés pourraient intervenir dans les phases de replication ou dans la production de particules virales infectieuses du VIH.

### IX - Mise en évidence de la molécule naturelle LAG-3

Des anticorps de lapin anti-LAG-3 ont été obtenus après injections répétées à des lapins d'un peptide synthétique couplé à la KLH. Ce peptide comprend 30 aminoacides formant la séquence représentée sur la séquence SEQ N° 3 inclus dans la boucle additionnelle du domaine V de LAG-3.

Après marquage à l'¹²⁵I des protéines de membrane de cellules T activées par la phytohémaglutinine, immunoprécipitation par les anticorps de lapin spécifiques et purification sur gel de SDS - polyacrilamide, une seule bande d'environ 55 kDaltons a été détectée en conditions réduite et non réduite (Fig. 7). Cette masse moléculaire observée d'environ 55 kDaltons pourrait correspondre à la masse moléculaire dérivée de l'analyse de la translation de la protéine LAG-3 (51 kDaltons) à laquelle s'ajoutent environ 5kDaltons dus à la présence de sucres (notamment sur certains des 4 sites de N-glycosylation), faisant de la protéine LAG-3 naturelle une glycoprotéine membranaire.

La protéine LAG-3 existe donc sous forme d'une chaîne unique, probablement glycosylée, à la surface des cellules T.

### X - Transcription in vitro et traduction du gène LAG-3

Afin de déterminer de façon définitive la région codante et la capacité d'être traduit de l'ARNm dérivé par transcription de la séquence FDC, nous avons transcrit les 2 brins de l'ADNc FDC (fragment Eco 47 III - Bgl II 209-1829 bp) en utilisant soit la T3, soit la T7 ARN polymérase et comme substrat le clone FDC dans le vecteur pBS. Les deux préparations d'ARN ont ensuite été traduites in vitro en utilisant un extrait de réticulocytes de lapin en présence de méthionine ³⁵S. L'ARN sens contenant la queue poly-A du côté 3' a été traduit en une protéine de masse moléculaire d'environ 55.000 daltons détectable en autoradiographie après migration sur gel de type SDS-PAGE. Cette masse moléculaire estimée est très semblable à la masse moléculaire théorique de 54457 correspondant aux 498 acides aminés du polypeptide LAG-3 avec un signal peptide intact, non clivé. Aucun produit de translation supérieur à 20000 daltons n'a pu être détecté en utilisant l'ARN anti-sens LAG-3 comme substrat dans la réaction avec l'extrait de réticulocyte de lapin.

### XI - Expression d'une protéine LAG-3 recombinante transmembranaire (LAG 3-C) et d'une protéine LAG-3 recombinante soluble (LAG 3-S)

Nous avons employé un système utilisant un vecteur de type "baculovirus". Ce système permet de produire des protéines étrangères aux cellules (cellules SF9) d'un insecte (Spodoptera Frugiperda) en utilisant une recombinaison in vivo entre un vecteur de transfert (plasmide p PVL 941) qui contient le gène étranger d'une part et d'autre part, le génome d'un virus (Autographa Californica Nuclear Polyhedrosis virus AcMNPV). Ce génome du virus est placé sous le contrôle fort du promoteur du gène de la protéine appelée polyhédrine. Ce système a été décrit par Luckow (21) après transfection du plasmide recombinant et du génome du virus; on sélectionne par purifications successives (criblage des recombinants) les cellules SF9 permettant d'obtenir une production de protéine recombinante importante. Cette protéine est normalement clivée (le peptide signal hydrophobe est enlevé à l'intérieur de la cellule) et glycosylée (au moins partiellement).

### 1) Construction du vecteur de transfert

### a) Préparation du vecteur

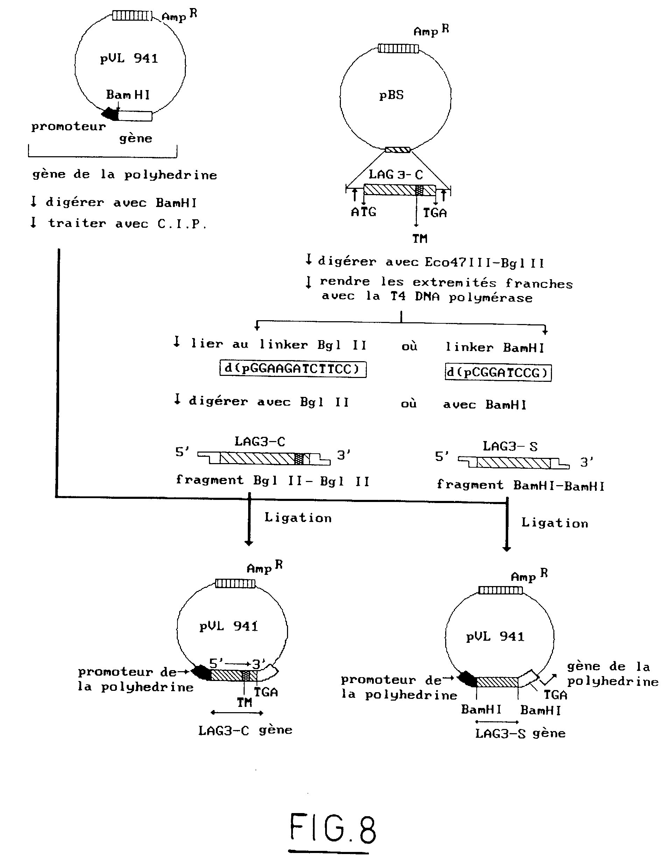

Le schéma de préparation du vecteur est représenté sur la Fig. 8.

Le vecteur PVL941 (accessible après du Dr. Max SUMMERS, Université du texas, U.S.A.) a été coupé par l'enzyme de restriction BAMHI puis déphosphorylée par l'enzyme phosphatase de l'intestin de veau ("Calf Intestine Phosphatase"). Ceci a été réalisé afin d'éviter l'autoligation du vecteur sur lui-même.

Nous avons ensuite préparé, à partir du clone d'ADNc FDC un fragment de 1620 paires de base correspondant à une digestion par les enzymes ECO47 III (position 209 de FDC) et BglII (position 1829 de FDC). Les extrémités coupées, d'une part par l'enzyme Eco47III et d'autre part par l'enzyme BglII ont été rendues de type bout franc par une réaction de synthèse avec une ADN polymérase de type Klenow (T4 DNA polymérase). On a ensuite lié un oligonucléotide double brin de type "linker" contenant un site de restriction BglII, au fragment FDC Eco47III BglII pour créer la construction LAG 3-C (C pour complet) et un linker contenant un site BamHI pour créer la construction LAG 3-S (S pour soluble). Après ligation, on a digéré avec un excès d'enzyme de restriction de type Bgl II (pour la construction LAG 3-C) ou BamHI (pour la construction LAG 3-S) puis purifié par électrophorèse sur gel les fragments correspondant aux 2 constructions. La dernière étape a consisté à lier le fragment LAG 3-C BglII ou le fragment LAG 3-S BamHI au vecteur PVL 941-BamHI.

### b) Sélection et amplification des vecteurs recombinants

Des bactéries JM109 compétentes ont été transformées avec le vecteur de transfert recombinant contenant l'une ou l'autre des constructions. Des colonies résistantes à l'ampicilline ont été mises en culture puis l'ADN plasmidique contenu dans ces bactéries a été purifié; on a ainsi obtenu un certain nombre de clones contenant le vecteur de transfert et on a sélectionné des clones comprenant le fragment LAG 3-C ou LAG 3-S dans la bonne direction. Afin d'obtenir le plasmide recombiné à l'état pur, susceptible d'être utilisé dans les expériences de transfection, le clone de bactéries ainsi obtenu a été mis en culture dans 500 ml de milieu avec ampicilline puis le plasmide a été purifié sur gradient de chlorure de césium.

### c) Purification d'ADN génomique du virus

Ceci a été fait selon la méthode décrite dans "A manual of methods for Baculovirus vectors and insect cell culture procedures" fourni par le Dr. Max SUMMERS de l'Université du Texas, U.S.A.

### d) Transfection des cellules avec le vecteur recombinant contenant l'insert LAG 3-C ou LAG 3-S et le génome du virus.

Il s'agit d'une co-transfection de cellules SF9 avec, d'une part, le vecteur recombinant purifié et, d'autre part, le génome du virus par la méthode du chlorure de calcium. Ceci a été fait selon les conditions du manuel référé en c).

### e) Sélection des virus recombinés

5 jours après transfection, les surnageants de cellules SF9 ont été recueillis puis testés. Ces tests sont effectués en infectant de nouvelles cellules SF9 avec des dilutions successives de ce surnageant de culture primaire. On considère tout d'abord qu'il y a 10⁷ pfu/ml (pfu = "plaque forming units") et on fait des dilutions successives de façon à obtenir entre 100 et 1 pfu/ml. Au bout de 3 jours, les cellules SF9 ainsi infectées sont testées par technique d'hybridation de type "dot blot". Les cellules sont lysées au NaOH, transférées sur nylon et hybridées avec une sonde correspondant au fragement FDC de 1871 paires de base. Après lavage et autoradiographie, les puits positifs sont repérés et on garde des puits correspondant aux dilutions les plus fortes. Cette technique de criblage est faite une deuxième puis une troisième fois. Lors du troisième criblage, on vérifie que les points donnant un signal positif en hybridation de type "dot blot" ne contiennent pas de cellules SF9 comprenant des inclusions. Ces inclusions correspondent à la sécrétion d'une protéine, la polyhédrine, produite après infection de cellules SF9 par un virus sauvage non recombiné. Ce dernier point a été aussi vérifié, non plus par lecture directe de la plaque, mais par une technique de recueil des cellules, d'étalement sur lame et de coloration par le May-Grünwald-Giemsa.

### f) Détection de la protéine recombinante LAG 3-C et LAG 3-S.

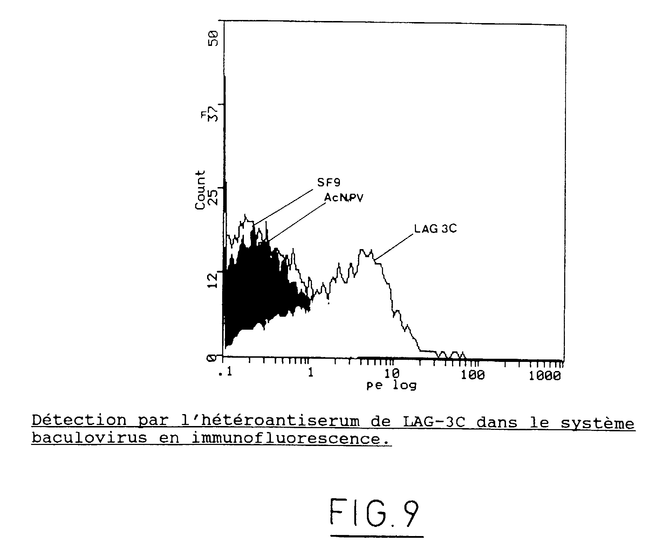

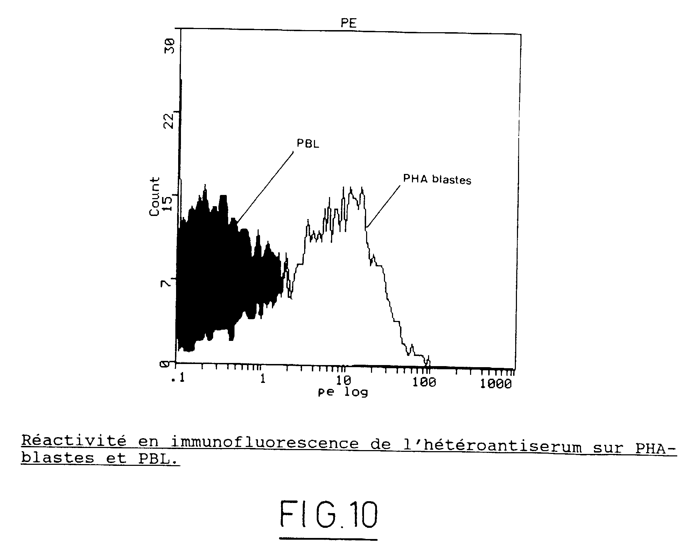

Des cellules SF9 infectées par le clone recombinant de virus contenant le fragment LAG 3-C ont été obtenus au 5^{e} jour de la culture après une infection correspondant à 0,1 pfu/cellules. Ces cellules SF9 expriment à la surface la molécule LAG-3 recombinante comme le montre la réactivité de l'anticorps de lapin spécifique en immunofluorescence, en comparant avec la réactivité obtenue avec des cellules SF9 non infectées ou infectées par un virus sauvage AcNPV (Fig. 9). De plus la réactivité du sérum de lapin spécifique de LAG-3 sur les cellules SF9 exprimant LAG-3 a été comparée à la réactivité obtenue sur des lymphocytes T activés par la phytohémaglutinine (PHA-blastes). Les histogrammes obtenus sont semblables et indiquent donc qu'un nombre comparable de molécules LAG-3 recombinantes (Fig. 9) est exprimé à la surface des cellules SF9 par rapport aux molécules naturelles LAG-3 exprimées à la surface des lymphocytes activés (Fig. 10).

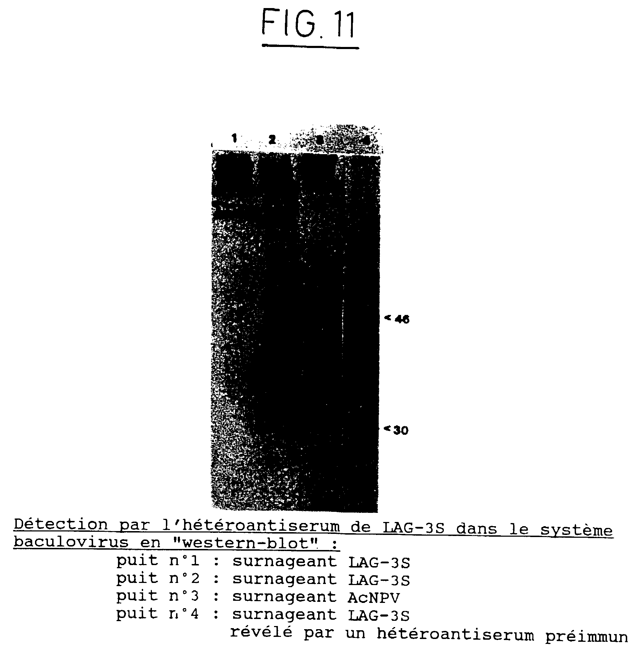

Des surnageants de cellules SF9 infectées par le clone recombinant de virus contenant le fragment LAG 3-S ont été obtenus au 5^{e} jour de la culture après une infection correspondant à 0,1 pfu/cellule. Un surnageant a été testé par la technique dite du "Western blot" avec les anticorps anti-peptidiques anti-boucle du domaine V décrits chapitre IX. On a ainsi obtenu un signal net correspondant à une protéine d'environ 45 kd après révélation avec des anticorps de chèvre anti-lapin marqués à la peroxydase (figure 11).

Cette masse moléculaire correspond bien à la masse attendue de la protéine de fusion LAG 3-S Eco47 III - BamHI (38038 K daltons) après glycosylation dans les cellules SF9.

La structure de la partie codante pour LAG-3 S (SEQ ID N° 5) montre que les trois premiers domaines de LAG-3 (en amont du site BamHI interne) ont été fusionnés à un segment nucléotidique de 56 paires de base du gène de la polyhédrine en aval du site BamHI. Au total, après clivage du peptide signal de 28 acides aminés, la protéine de fusion comprend 352 acides aminés, 335 correspondant à LAG-3 et 17 provenant d'un des cadres de lecture du gêne de la polyhédrine.

### REFERENCES

1. Nowill, A. et al., J. Exp. Med. 163, 1601.
2. Maniatis, T. et al., 1982. Molecular cloning : A laboratory manual, Cold spring harbor laboratory New York.
3. Mechler, B. et al., J. Cell Biol. 88, 29 (1981).
4. Aviv et al., Proc. Natl. Acad. Sci. USA 69 : 1408.
5. Triebel, F. et al., Eur. J. Immunol. 17, 1209.
6. Gubler, U. et al., Gene. 25, 263.
7. Davis, M.M. et al., Proc. Natl. Acad. Sci. USA. 81:2194.
8. Huynh, T.V. et al., DNA cloning : A practical approach. 49-78, D. Glover Editor. IRL Press. Oxford. United Kingdom.
9. Sanger, F. et al., Proc. Natl. Acad. Sci. USA 75, 5463.
10. Dariavach, P. et al., Proc. Natl. Acad. Sci. USA. 84, 9074.
11. Feinberg, A.P. et al., Anal. Biochem. 132, 6.
12. Amzel, L.M. et al., Ann. Rev. Biochem 48, 961 (1979).
13. Williams, A.F. Immunol. Today 8, 298 (1987).
14. Lesk, A.M. & Chothia, C.J. Mol. Biol. 160, 325 (1982).
15. Santoni, M.J. et al. EMBO J. 8, 395 (1989).
16. Kirszbaum, L. et al., J. Immunol. 142, 3931 (1989).
17. Ruoslahti, E. et al., M.D. Cell 44, 517 (1986).
18. Dayhoff, M.O. et al., Enzymol. 91, 524 (1983).
19. Williams, A.F. et al., Ann. Rev. Immunol. 6, 381.
20. Maddon, P.J. et al. Proc. Natl. Acad. Sci. USA 84, 9155 (1987).
21. Luckow, V.A. et al., Bio/Technology, 6:47.
22. Ryu S.E. et al., Nature, 348, 419.
23. Wang J. et al., Nature, 348, 411
24. Byrn R.A. et al., Nature 344, 667
25. Staunton D.E. et al., Cell. 61, 243
26. Wegner C.D. et al., Science 247, 456
27. Marlin S.D. et al., Nature 344, 70
28. Triebel F. et al;, J. Exp. Med., 171, 1393
29. Hart E.C. et al., Science, 240, 488
30. Yourno J. et al., AIDS Res. Hum. Retroviruses 4:165-173(1988).
31. Ratner L. et al., Nature 313:277-284 (1985).

| Symboles des acides aminés | | |
|---|---|---|
| A | Ala | alanine |
| C | Cys | cytéine |
| D | Asp | acide aspartique |
| E | Glu | acide glutamique |
| F | Phe | phénylalanine |
| G | Gly | glycine |
| H | His | histidine |
| I | Ils | isoleucine |
| K | Lys | lysine |
| L | Leu | leucine |
| M | Met | méthionine |
| N | Asn | asparagine |
| P | Pro | proline |
| Q | Gln | glutamine |
| R | Arg | arginine |
| S | Ser | serine |
| T | Thr | thréonine |
| V | Val | valine |
| W | Trp | tryptophane |
| Y | Tyr | tyrosine |

### LISTE DES SEQUENCES

I - INFORMATION GENERALE
   (1) DEMANDEUR : INSTITUT GUSTAVE ROUSSY
   (2) TITRE DE L'INVENTION :
      Nouvelles protéines produites par les lymphocytes humains, séquence d'ADN codant pour ces protéines et applications pharmaceutiques et biologiques
   (3) NOMBRE DE SEQUENCES : 5
II - INFORMATION POUR SEQ ID N° 1
   CARACTERISTIQUES DES SEQUENCES
   TYPE : nucléotide et sa protéine correspondante
   LONGUEUR : 1871 paires de bases
   NOMBRE DE BRINS : simple
   CONFIGURATION : linéaire
   TYPE DE MOLECULE : ADNc pour ARNm
   ORIGINE
   ORGANISME : humain
   LIGNEE CELLULAIRE : lymphocytes humains
   CARACTERISTIQUE
   NOM DE L'ADN : FDC (gène LAG-3)
   NOM DE LA PROTEINE : LAG-3
   DESCRIPTION DE LA SEQUENCE 
TCAGGCTGCC TGATCTGCCC AGCTTTCCAGC TTTCCTCTG GATTCCGGCC TCTGGTCATC 60

CCTCCCCACC CTCTCTCCAA GGCCCTCTCC TGGTCTCCCT TCTTCTAGAA CCCCTTCCTC 120

CACCTCCCTC TCTGCAGAAC TTCTCCTTTA CCCCCCACCC CCCACCACTG CCCCCTTTCC 180

TTTTCTGAC CTCCTTTTG GAGGGCTCAG CGCTGCCCA GACCATAGG AGAG ATG TGG GAG 239
Met Trp Glu
-28

GCT CAG TTC CTG GGC TTG CTG TTT CTG CAG CCG CTT TGG GTG GCT CCA 287
Ala Gln Phe Leu Gly Leu Leu Phe Leu Gln Pro Leu Trp Val Ala Pro
-25 -20 -15 -10

GTG AAG CCT CTC CAG CCA GGG GCT GAG GTC CCG GTG GTG TGG GCC CAG 335
Val Lys Pro Leu Gln Pro Gly Ala Glu Val Pro Val Val Trp Ala Gln
-5 1 5

GAG GGG GCT CCT GCC CAG CTC CCC TGC AGC CCC ACA ATC CCC CTC CAG 383
Glu Gly Ala Pro Ala Gln Leu Pro Cys Ser Pro Thr Ile Pro Leu Gln
10 15 20

GAT CTC AGC CTT CTG CGA AGA GCA GGG GTC ACT TGG CAG CAT CAG CCA 431
Asp Leu Ser Leu Leu Arg Arg Ala Gly Val Thr Trp Gln His Gln Pro
25 30 35

GAC AGT GGC CCG CCC GCT GCC GCC CCC GGC CAT CCC CTG GCC CCC GGC 479
Asp Ser Gly Pro Pro Ala Ala Ala Pro Gly His Pro Leu Ala Pro Gly
40 45 50 55

CCT CAC CCG GCG GCG CCC TCC TCC TGG GGG CCC AGG CCC CGC CGC TAC 527
Pro His Pro Ala Ala Pro Ser Ser Trp Gly Pro Arg Pro Arg Arg Tyr
60 65 70

ACG GTG CTG AGC GTG GGT CCC GGA GGC CTG CGC AGC GGG AGG CTG CCC 575
Thr Val Leu Ser Val Gly Pro Gly Gly Leu Arg Ser Gly Arg Leu Pro
75 80 85

CTG CAG CCC CGC GTC CAG CTG GAT GAG CGC GGC CGG CAG CGC GGG GAC 623
Leu Gln Pro Arg Val Gln Leu Asp Glu Arg Gly Arg Gln Arg Gly Asp
90 95 100

TTC TCG CTA TGG CTG CGC CCA GCC CGG CGC GCG GAC GCC GGC GAG TAC 671
Phe Ser Leu Trp Leu Arg Pro Ala Arg Arg Ala Asp Ala Gly Glu Tyr
105 110 115

CGC GCC GCG GTG CAC CTC AGG GAC CGC GCC CTC TCC TGC CGC CTC CGT 719
Arg Ala Ala Val His Leu Arg Asp Arg Ala Leu Ser Cys Arg Leu Arg
120 125 130 135

CTG CGC CTG GGC CAG GCC TCG ATG ACT GCC AGC CCC CCA GGA TCT CTC 767
Leu Arg Leu Gly Gln Ala Ser Met Thr Ala Ser Pro Pro Gly Ser Leu
140 145 150

AGA GCC TCC GAC TGG GTC ATT TTG AAC TGC TCC TTC AGC CGC CCT GAC 815
Arg Ala Ser Asp Trp Val Ile Leu Asn Cys Ser Phe Ser Arg Pro Asp
155 160 165

CGC CCA GCC TCT GTG CAT TGG TTC CGG AAC CGG GGC CAG GGC CGA GTC 863
Arg Pro Ala Ser Val His Trp Phe Arg Asn Arg Gly Gln Gly Arg Val
170 175 180
 
CCT GTC CGG GAG TCC CCC CAT CAC CAC TTA GCG GAA AGC TTC CTC TTC 911
Pro Val Arg Glu Ser Pro His His His Leu Ala Glu Ser Phe Leu Phe
185 190 195

CTG CCC CAA GTC AGC CCC ATG GAC TCT GGG CCC TGG GGC TGC ATC CTC 959
Leu Pro Gln Val Ser Pro Met Asp Ser Gly Pro Trp Gly Cys Ile Leu
200 205 210 215

ACC TAC AGA GAT GGC TTC AAC GTC TCC ATC ATG TAT AAC CTC ACT GTT 1007
Thr Tyr Arg Asp Gly Phe Asn Val Ser Ile Met Tyr Asn Leu Thr Val
220 225 230

CTG GGT CTG GAG CCC CCA ACT CCC TTG ACA GTG TAC GCT GGA GCA GGT 1055
Leu Gly Leu Glu Pro Pro Thr Pro Leu Thr Val Tyr Ala Gly Ala Gly
235 240 245

TCC AGG GTG GGG CTG CCC TGC CGC CTG CCT GCT GGT GTG GGG ACC CGG 1103
Ser Arg Val Gly Leu Pro Cys Arg Leu Pro Ala Gly Val Gly Thr Arg
250 255 260

TCT TTC CTC ACT GCC AAG TGG ACT CCT CCT GGG GGA GGC CCT GAC CTC 1151
Ser Phe Leu Thr Ala Lys Trp Thr Pro Pro Gly Gly Gly Pro Asp Leu
265 270 275

CTG GTG ACT GGA GAC AAT GGC GAC TTT ACC CTT CGA CTA GAG GAT GTG 1199
Leu Val Thr Gly Asp Asn Gly Asp Phe Thr Leu Arg Leu Glu Asp Val
280 285 290 295

AGC CAG GCC CAG GCT GGG ACC TAC ACC TGC CAT ATC CAT CTG CAG GAA 1247
Ser Gln Ala Gln Ala Gly Thr Tyr Thr Cys His Ile His Leu Gln Glu
300 305 310

CAG CAG CTC AAT GCC ACT GTC ACA TTG GCA ATC ATC ACA GTG ACT CCC 1295
Gln Gln Leu Asn Ala Thr Val Thr Leu Ala Ile Ile Thr Val Thr Pro
315 320 325

AAA TCC TTT GGG TCA CCT GGA TCC CTG GGG AAG CTG CTT TGT GAG GTG 1343
Lys Ser Phe Gly Ser Pro Gly Ser Leu Gly Lys Leu Leu Cys Glu Val
330 335 340

ACT CCA GTA TCT GGA CAA GAA CGC TTT GTG TGG AGC TCT CTG GAC ACC 1391
Thr Pro Val Ser Gly Gln Glu Arg Phe Val Trp Ser Ser Leu Asp Thr
345 350 355

CCA TCC CAG AGG AGT TTC TCA GGA CCT TGG CTG GAG GCA CAG GAG GCC 1439
Pro Ser Gln Arg Ser Phe Ser Gly Pro Trp Leu Glu Ala Gln Glu Ala
360 365 370 375

CAG CTC CTT TCC CAG CCT TGG CAA TGC CAG CTG TAC CAG GGG GAG AGG 1487
Gln Leu Leu Ser Gln Pro Trp Gln Cys Gln Leu Tyr Gln Gly Glu Arg
380 385 390

CTT CTT GGA GCA GCA GTG TAC TTC ACA GAG CTG TCT AGC CCA GGT GCC 1535
Leu Leu Gly Ala Ala Val Tyr Phe Thr Glu Leu Ser Ser Pro Gly Ala
395 400 405

CAA CGC TCT GGG AGA GCC CCA GGT GCC CTC CCA GCA GGC CAC CTC CTG 1583
Gln Arg Ser Gly Arg Ala Pro Gly Ala Leu Pro Ala Gly His Leu Leu
410 415 420

CTG TTT CTC ACC CTT GGT GTC CTT TCT CTG CTC CTT TTG GTG ACT GGA 1631
Leu Phe Leu Thr Leu Gly Val Leu Ser Leu Leu Leu Leu Val Thr Gly
425 430 435

GCC TTT GGC TTT CAC CTT TGG AGA AGA CAG TGG CGA CCA AGA CGA TTT 1679
 
Ala Phe Gly Phe His Leu Trp Arg Arg Gln Trp Arg Pro Arg Arg Phe
440                 445                 450                 455

TCT GCC TTA GAG CAA GGG ATT CAC CCT CGC AGG CTC AGA GCA AGA          1724
Ser Ala Leu Glu Gln Gly Ile His Pro Arg Arg Leu Arg Ala Arg
                460                 465                 470

TAGAGGAGCT GGAGCAAGAA CCGGAGCCGG AGCCGGAGCC GGAACCGGAG CCCGAGCCCG    1784

AGCCCGAGCC GGAGCAGCTC TGACCTGGAG CTGAGGCAGC CAGCAGATCT CAGCAGCCCA    1744

GTCCAAATAA ACGTCCTGTC TAGCAGC                                        1871
III - INFORMATION POUR SEQ ID N° 2
   TYPE : protéine
   DESCRIPTION DE LA SEQUENCE 
Xaa Val Pro Val Val Trp Ala Gln
-5 1 5

Glu Gly Ala Pro Ala Gln Leu Pro Cys Ser Pro Thr Ile Pro Leu Gln
10 15 20

Asp Leu Ser Leu Leu Arg Arg Ala Gly Val Thr Trp Gln His Gln Pro
25 30 35

Asp Ser Gly Pro Pro Ala Ala Ala Pro Gly His Pro Leu Ala Pro Gly
40 45 50 55

Pro His Pro Ala Ala Pro Ser Ser Trp Gly Pro Arg Pro Arg Arg Tyr
60 65 70

Thr Val Leu Ser Val Gly Pro Gly Gly Leu Arg Ser Gly Arg Leu Pro
75 80 85

Leu Gln Pro Arg Val Gln Leu Asp Glu Arg Gly Arg Gln Arg Gly Asp
90 95 100

Phe Ser Leu Trp Leu Arg Pro Ala Arg Arg Ala Asp Ala Gly Glu Tyr
105 110 115

Arg Ala Ala Val His Leu Arg Asp Arg Ala Leu Ser Cys Arg Leu Arg
120 125 130 135

Leu Arg Leu Gly Gln Ala Ser Met Thr Ala Ser Pro Pro Gly Ser Leu
140 145 150

Arg Ala Ser Asp Trp Val Ile Leu Asn Cys Ser Phe Ser Arg Pro Asp
155 160 165

Arg Pro Ala Ser Val His Trp Phe Arg Asn Arg Gly Gln Gly Arg Val
170 175 180

Pro Val Arg Glu Ser Pro His His His Leu Ala Glu Ser Phe Leu Phe
185 190 195

Leu Pro Gln Val Ser Pro Met Asp Ser Gly Pro Trp Gly Cys Ile Leu
200 205 210 215
 
Thr Tyr Arg Asp Gly Phe Asn Val Ser Ile Met Tyr Asn Leu Thr Val
220 225 230

Leu Gly Leu Glu Pro Pro Thr Pro Leu Thr Val Tyr Ala Gly Ala Gly
235 240 245

Ser Arg Val Gly Leu Pro Cys Arg Leu Pro Ala Gly Val Gly Thr Arg
250 255 260

Ser Phe Leu Thr Ala Lys Trp Thr Pro Pro Gly Gly Gly Pro Asp Leu
265 270 275

Leu Val Thr Gly Asp Asn Gly Asp Phe Thr Leu Arg Leu Glu Asp Val
280 285 290 295

Ser Gln Ala Gln Ala Gly Thr Tyr Thr Cys His Ile His Leu Gln Glu
300 305 310

Gln Gln Leu Asn Ala Thr Val Thr Leu Ala Ile Ile Thr Val Thr Pro
315 320 325

Lys Ser Phe Gly Ser Pro Gly Ser Leu Gly Lys Leu Leu Cys Glu Val
330 335 340

Thr Pro Val Ser Gly Gln Glu Arg Phe Val Trp Ser Ser Leu Asp Thr
345 350 355

Pro Ser Gln Arg Ser Phe Ser Gly Pro Trp Leu Glu Ala Gln Glu Ala
360 365 370 375

Gln Leu Leu Ser Gln Pro Trp Gln Cys Gln Leu Tyr Gln Gly Glu Arg
380 385 390

Leu Leu Gly Ala Ala Val Tyr Phe Thr Glu Leu Ser Ser Pro Gly Ala
395 400 405

Gln Arg Ser Gly Arg Ala Pro Gly Ala Leu Pro Ala Gly His Leu Leu
410 415 420

Leu Phe Leu Thr Leu Gly Val Leu Ser Leu Leu Leu Leu Val Thr Gly
425 430 435

Ala Phe Gly Phe His Leu Trp Arg Arg Gln Trp Arg Pro Arg Arg Phe
440 445 450 455

Ser Ala Leu Glu Gln Gly Ile His Pro Arg Arg Leu Arg Ala Arg
460 465 470
IV - INFORMATION POUR SEQ ID N° 3
   TYPE : séquence peptidique
   DESCRIPTION DE LA SEQUENCE 
Gly Pro Pro Ala Ala Ala Pro Gly His Pro
Leu Ala Pro Gly Pro His Pro Ala Ala Pro
Ser Ser Trp Gly Pro Arg Pro Arg Arg Tyr
V - INFORMATION POUR SEQ ID N° 4
   CARACTERISTIQUES DE LA SEQUENCE
   TYPE DE SEQUENCE : nucléotide
   NOMBRE DE BRINS : simple
   CONFIGURATION : linéaire
   TYPE DE MOLECULE : ADN génomique 
CCTGCAATGT CATTCTTTGA GCTCAGTTCC TCATCTCTGT CATGGAGAGC ATTAGATTTC 60

ATGAATTCAT ACTAAGTGTC CAATACAGTG CTTAGCACGT AATGAAGCCT CAATACAATG 120

TAGTTATTCT CCATGCCCCA CAAAGCTGCA TGCCTAGCCT CAGACCTACC ATTTTTTGGG 180

GTGCAGTAAG GCTTCCTGTC CACCATGTTC CCAGGGACAT TGTACTGATG GGTGGAAAGG 240

CAGGTCTAAA GGGGTCACGA AGTTCTGGGA GGTTAAGGGA ACGAGGAAGG AGATTGAGCA 300

ACAAGGAAAG AGCTTGCCAA GAAGGAGGTG TGAATATTGG GACTGAGGAG GCAGCTTAGA 360

GATGGGCAAG GGGGCAGTTC CAGGCAGAAA TGGTTCGTGG AGGCAGAAGG TCCCTGGGAG 420

AGGGAGCAGT CTGGAGGGTG GGGCAGGGGC GAGGAGGGGG AGGTGGGGAG ACCCAGGACT 480

GAGGAAGTAA ACAAGGGGAG CGCCACCACA GAGGTGGAGA GGTGGAGGGT GCTGCTGCTG 540

GGAATCAACC CCCTCAGACT TTCCACTGCG AAGCGAAACC GTAAGCCCTG GGGTGCGGGG 600

GGCGGGCCGG GAGGAGGGGA AGTGGGGAAG GTGGAGGGAA GGCCGGGCAC AGGGGTGAAG 660

GCCCAGAGAC CAGCAGAACG GCATCCCAGC CACGACGGCC ACTTTGCTCT GTCTGCTGTC 720

CGCCACGGCC CTGCTCTGTT CCCTGGGACA CCCCCGCCCC CACCTCCTCA GGCTGCCTGA 780

TCTGCCCAGC TTTCCAGCTT TCCTCTGGAT TCCGGCCTCT GGTCATCCCT CCCCACCCTC 840

TCTCCAAGGC CCTCTCCTGG TCTCCCTTCT TCTAGAACCC CTTCCTCCAC CTCCCTCTCT 900

GCAGAACTTC TCCTTTCCCC CCACCCCCCA CCACTGCCCC CTTTCCTTTT CTGACCTCCT 960

TTTGGAGGGC TCAGCGCTGC CCAGACCATA GGAGAGATG 999
VI - INFORMATION POUR SEQ ID N° 5
   CARACTERISTIQUE DES SEQUENCES
   TYPE : nucléotide et sa protéine correspondante
   NOMBRE DE BRINS : simple
   CONFIGURATION : linéaire

GCTGCCCAGA CCATAGGAGA G ATG TGG GAG
Met Trp Glu
-28

GCT CAG TTC CTG GGC TTG CTG TTT CTG CAG CCG CTT TGG GTG GCT CCA 78
Ala Gln Phe Leu Gly Leu Leu Phe Leu Gln Pro Leu Trp Val Ala Pro
-25 -20 -15 -10

GTG AAG CCT CTC CAG CCA GGG GCT GAG GTC CCG GTG GTG TGG GCC CAG 126
Val Lys Pro Leu Gln Pro Gly Ala Glu Val Pro Val Val Trp Ala Gln
-5 1 5

GAG GGG GCT CCT GCC CAG CTC CCC TGC AGC CCC ACA ATC CCC CTC CAG 174
Glu Gly Ala Pro Ala Gln Leu Pro Cys Ser Pro Thr Ile Pro Leu Gln
10 15 20

GAT CTC AGC CTT CTG CGA AGA GCA GGG GTC ACT TGG CAG CAT CAG CCA 222
Asp Leu Ser Leu Leu Arg Arg Ala Gly Val Thr Trp Gln His Gln Pro
25 30 35

GAC AGT GGC CCG CCC GCT GCC GCC CCC GGC CAT CCC CTG GCC CCC GGC 270
Asp Ser Gly Pro Pro Ala Ala Ala Pro Gly His Pro Leu Ala Pro Gly
40 45 50 55

CCT CAC CCG GCG GCG CCC TCC TCC TGG GGG CCC AGG CCC CGC CGC TAC 318
Pro His Pro Ala Ala Pro Ser Ser Trp Gly Pro Arg Pro Arg Arg Tyr
60 65 70

ACG GTG CTG AGC GTG GGT CCC GGA GGC CTG CGC AGC GGG AGG CTG CCC 366
Thr Val Leu Ser Val Gly Pro Gly Gly Leu Arg Ser Gly Arg Leu Pro
75 80 85

CTG CAG CCC CGC GTC CAG CTG GAT GAG CGC GGC CGG CAG CGC GGG GAC 414
Leu Gln Pro Arg Val Gln Leu Asp Glu Arg Gly Arg Gln Arg Gly Asp
90 95 100

TTC TCG CTA TGG CTG CGC CCA GCC CGG CGC GCG GAC GCC GGC GAG TAC 462
Phe Ser Leu Trp Leu Arg Pro Ala Arg Arg Ala Asp Ala Gly Glu Tyr
105 110 115

CGC GCC GCG GTG CAC CTC AGG GAC CGC GCC CTC TCC TGC CGC CTC CGT 510
Arg Ala Ala Val His Leu Arg Asp Arg Ala Leu Ser Cys Arg Leu Arg
120 125 130 135

CTG CGC CTG GGC CAG GCC TCG ATG ACT GCC AGC CCC CCA GGA TCT CTC 558
Leu Arg Leu Gly Gln Ala Ser Met Thr Ala Ser Pro Pro Gly Ser Leu
140 145 150

AGA GCC TCC GAC TGG GTC ATT TTG AAC TGC TCC TTC AGC CGC CCT GAC 606
Arg Ala Ser Asp Trp Val Ile Leu Asn Cys Ser Phe Ser Arg Pro Asp
155 160 165

CGC CCA GCC TCT GTG CAT TGG TTC CGG AAC CGG GGC CAG GGC CGA GTC 654
Arg Pro Ala Ser Val His Trp Phe Arg Asn Arg Gly Gln Gly Arg Val
170 175 180

CCT GTC CGG GAG TCC CCC CAT CAC CAC TTA GCG GAA AGC TTC CTC TTC 702
Pro Val Arg Glu Ser Pro His His His Leu Ala Glu Ser Phe Leu Phe
185 190 195

CTG CCC CAA GTC AGC CCC ATG GAC TCT GGG CCC TGG GGC TGC ATC CTC 750

Leu Pro Gln Val Ser Pro Met Asp Ser Gly Pro Trp Gly Cys Ile Leu
200 205 210 215

ACC TAC AGA GAT GGC TTC AAC GTC TCC ATC ATG TAT AAC CTC ACT GTT 798
Thr Tyr Arg Asp Gly Phe Asn Val Ser Ile Met Tyr Asn Leu Thr Val
220 225 230

CTG GGT CTG GAG CCC CCA ACT CCC TTG ACA GTG TAC GCT GGA GCA GGT 846
Leu Gly Leu Glu Pro Pro Thr Pro Leu Thr Val Tyr Ala Gly Ala Gly
235 240 245

TCC AGG GTG GGG CTG CCC TGC CGC CTG CCT GCT GGT GTG GGG ACC CGG 894
Ser Arg Val Gly Leu Pro Cys Arg Leu Pro Ala Gly Val Gly Thr Arg
250 255 260

TCT TTC CTC ACT GCC AAG TGG ACT CCT CCT GGG GGA GGC CCT GAC CTC 942
Ser Phe Leu Thr Ala Lys Trp Thr Pro Pro Gly Gly Gly Pro Asp Leu
265 270 275

CTG GTG ACT GGA GAC AAT GGC GAC TTT ACC CTT CGA CTA GAG GAT GTG 9[?][?]
Leu Val Thr Gly Asp Asn Gly Asp Phe Thr Leu Arg Leu Glu Asp Val
280 285 290 295

AGC CAG GCC CAG GCT GGG ACC TAC ACC TGC CAT ATC CAT CTG CAG GAA 1038
Ser Gln Ala Gln Ala Gly Thr Tyr Thr Cys His Ile His Leu Gln Glu
300 305 310

CAG CAG CTC AAT GCC ACT GTC ACA TTG GCA ATC ATC ACA GTG ACT CCC 1086
Gln Gln Leu Asn Ala Thr Val Thr Leu Ala Ile Ile Thr Val Thr Pro
315 320 325

AAA TCC TTT GGG TCA CCT GGA TCC TTT CCT GGG ACC CGG CAA GAA CCA 1134
Lys Ser Phe Gly Ser Pro Gly Ser Phe Pro Gly Thr Arg Gln Glu Pro
330 335 340

AAA ACT CAC TCT CTT CAA GGA AAT CCG TAA 1164
Lys Thr His Ser Leu Gln Gly Asn Pro
345 350

## Revendications

1. Protéines constituées par la séquence peptidique représentée par la séquence SEQ ID N° 1 ou ses fractions et les séquences qui en diffèrent par un ou plusieurs amino-acides et qui se lient aux anticorps monoclonaux spécifiques d'une protéine représentée par la séquence SEQ ID N° 1.

2. Protéine selon la revendication 1, constituée par la séquence peptidique représentée par la séquence SEQ ID N° 2 dans laquelle Xaa est choisi parmi H et un fragment de la séquence -28 à -1 de la séquence protéique représentée par la séquence SEQ ID N° 1, et les séquences qui en diffèrent par un ou plusieurs amino-acides et qui se lient aux anticorps monoclonaux spécifiques d'une protéine représentée par la séquence SEQ ID N° 1.

3. Fraction soluble d'une protéine selon la revendication 1 ou la revendication 2, comprenant au moins l'un des quatre domaines extracellulaires de type immunoglobuline de ladite protéine.

4. Fraction soluble d'une protéine selon la revendication 1, comprenant la région intracytoplasmique de ladite protéine.

5. Séquence peptidique immunogène comprenant la séquence représentée par la séquence SEQ ID N° 3 de la protéine selon la revendication 1 ou la revendication 2.

6. Protéines ou peptides selon l'une quelconque des revendications 1 à 5, comprenant un reste méthionine additionnel à l'extrémité -NH₂.

7. Séquence d'ADN codant pour une protéine selon l'une quelconque des revendications 1 à 6, ses variants alléliques ou des variants résultant de la dégénérescence du code génétique ou de mutations ponctuelles et codant pour une protéine selon l'une quelconque des revendications 1 à 6.

8. Séquence d'ADN selon la revendication 7, comprenant essentiellement une séquence correspondant à la séquence d'ADN représentée par la séquence SEQ ID N° 1.

9. Séquence d'ADN selon la revendication 7 ou la revendication 8, qui est une séquence d'ADNc.

10. Séquence d'ADN selon la revendication 7, qui est une séquence d'ADN génomique et qui comprend environ 6,6 kb et comporte 8 exons dont les premiers nucléotides sont respectivement ceux en position 1, 289, 437, 742, 1012, 1288, 1531 et 1662 de la séquence d'ADN selon la revendication 8.

11. Séquence d'ADN selon la revendication 10, caractérisée en ce que sa carte de restriction par les endonucléases Sp I, Xba I, Pst I, Sac I, Bam HI, Hind III, Eco RI est telle que représentée à la Fig. 2.

12. Séquence d'ADN capable de s'hybrider dans des conditions de stringence à une séquence d'ADN selon l'une quelconque des revendications 7 à 11.

13. Anticorps monoclonaux dirigés contre une protéine selon la revendication 1 ou la revendication 2 ou une séquence immunogène d'une telle protéine.

14. Anticorps monoclonaux selon la revendication 13, dirigés contre la séquence selon la revendication 5.

15. Fragments Fab, Fab', F(ab')₂ ou Fv d'anticorps monoclonaux selon la revendication 13.

16. Dérivés d'anticorps monoclonaux comprenant des anticorps monoclonaux selon la revendication 13 ou la revendication 14 ou des fragments selon la revendication 15 auxquels sont liés des marqueurs ou des molécules thérapeutiquement actives.

17. Hybridomes produisant des anticorps monoclonaux selon la revendication 13.

18. Composition thérapeutique comprenant des anticorps monoclonaux selon la revendication 13, des fragments d'anticorps monoclonaux selon la revendication 15 ou des dérivés d'anticorps monoclonaux selon la revendication 16.

19. Composition thérapeutique selon la revendication 18, dans laquelle les dérivés d'anticorps monoclonaux sont des anticorps monoclonaux ou des fragments de ces anticorps liés à une molécule cytotoxique ou à un radioisotope.

20. Procédé de dosage d'une protéine selon l'une quelconque des revendications 1 à 6, comprenant l'utilisation d'anticorps monoclonaux dirigés contre une protéine selon la revendication 1 ou la revendication 2 ou une séquence immunogène d'une telle protéine.

21. Vecteur d'expression comprenant une séquence d'ADN selon l'une quelconque des revendications 7 à 12.

22. Hôte transformé avec un vecteur selon la revendication 21.

23. Séquence d'ADN comprenant essentiellement comme séquence promotrice tout ou partie de la séquence représentée par la séquence SEQ ID N° 4.

24. Vecteur d'expression comprenant comme séquence promotrice une séquence selon la revendication 23.

25. Hôte transformé avec un vecteur selon la revendication 24.

26. Procédé de préparation d'une protéine, d'un fragment de protéine ou d'un peptide selon l'une quelconque des revendications 1 à 6, comprenant l'insertion d'une séquence d'ADN selon l'une quelconque des revendications 7 à 12 dans un vecteur d'expression, la transformation de cellules avec ce vecteur d'expression et la culture de ces cellules.

27. Procédé selon la revendication 26, dans lequel les cellules hôtes sont des cellules eucaryotes.

28. Procédé selon la revendication 26, dans lequel les cellules hôtes sont des cellules procaryotes.

29. Anticorps monoclonaux selon la revendication 13 ou protéines selon l'une quelconque des revendications 1 à 6, destinés à être utilisés comme médicament.

30. Utilisation d'anticorps monoclonaux selon la revendication 13 ou de protéines ou peptides selon l'une des revendications 1 à 6 pour la préparation d'un médicament destiné au traitement des affections humaines liées à l'infection par les virus du type VIH.

31. Utilisation d'anticorps monoclonaux selon la revendication 13 ou de protéines ou de peptides selon l'une quelconque des revendications 1 à 6 pour la préparation d'un médicament destiné au traitement de maladies faisant intervenir une interaction d'adhésion intercellulaire entre un ligand et la protéine selon la revendication 2.

32. Utilisation d'anticorps monoclonaux selon la revendication 13 ou de protéines ou de peptides selon l'une quelconque des revendications 1 à 6 pour la préparation d'un médicament destiné au traitement des maladies humaines causées par des virus se liant de façon spécifique à la protéine selon la revendication 2.

33. Composition pharmaceutique comprenant à titre de principe actif une protéine ou un peptide selon l'une quelconque des revendications 1 à 6.

## Claims

1. Proteins which consist of the peptide sequence represented by the sequence SEQ ID No. 1 or its fractions and the sequences which differ from it by one or more amino acids and which bind to the monoclonal antibodies which are specific for a protein represented by the sequence SEQ ID No. 1.

2. Protein according to Claim 1, which consists of the peptide sequence represented by the sequence SEQ ID No. 2 in which Xaa is selected from H and a fragment of the sequence -28 to -1 of the protein sequence represented by the sequence SEQ ID No. 1, and the sequences which differ from it by one or more amino acids and which bind to the monoclonal antibodies which are specific for a protein represented by the sequence SEQ ID No. 1.

3. Soluble fraction of a protein according to Claim 1 or Claim 2, which comprises at least one of the four immunoglobulin-type extracellular domains of the said protein.

4. Soluble fraction of a protein according to Claim 1, which comprises the intracytoplasmic region of the said protein.

5. Immunogenic peptide sequence which comprises the sequence represented by the sequence SEQ ID No. 3 of the protein according to Claim 1 or Claim 2.

6. Proteins or peptides according to any one of Claims 1 to 5, which comprise an additional methionine residue at the -NH₂ end.

7. DNA sequence which encodes a protein according to any one of Claims 1 to 6, its allelic variants or variants which result from the degeneracy of the genetic code or from point mutations and which encode a protein according to any one of Claims 1 to 6.

8. DNA sequence according to Claim 7, which essentially comprises a sequence which corresponds to the DNA sequence represented by the sequence SEQ ID No. 1.

9. DNA sequence according to Claim 7 or Claim 8, which is a cDNA sequence.

10. DNA sequence according to Claim 7, which is a genomic DNA sequence and which comprises approximately 6.6 kb and is made up of 8 exons, the first nucleotides of which are respectively those in positions 1, 289, 437, 742, 1012, 1288, 1531 and 1662 of the DNA sequence according to Claim 8.

11. DNA sequence according to Claim 10, characterized in that its restriction map constructed using the endonucleases Sp I, Xba I, Pst I, Sac I, Bam HI, Hind III and Eco RI is as depicted in Fig. 2.

12. DNA sequence which is able to hybridize, under stringent conditions, with a DNA sequence according to any one of Claims 7 to 11.

13. Monoclonal antibodies which are directed against a protein according to Claim 1 or Claim 2 or an immunogenic sequence of such a protein.

14. Monoclonal antibodies according to Claim 13, which are directed against the sequence according to Claim 5.

15. Fab, Fab', F(ab')₂ or Fv fragments of monoclonal antibodies according to Claim 13.

16. Derivatives of monoclonal antibodies which comprise monoclonal antibodies according to Claim 13 or Claim 14 or fragments according to Claim 15 to which therapeutically active molecules or labels are linked.

17. Hybridomas which produce monoclonal antibodies according to Claim 13.

18. Therapeutic composition which comprises monoclonal antibodies according to Claim 13, fragments of monoclonal antibodies according to Claim 15 or derivatives of monoclonal antibodies according to Claim 16.

19. Therapeutic composition according to Claim 18 in which the derivatives of monoclonal antibodies are monoclonal antibodies or fragments of these antibodies which are linked to a cytotoxic molecule or to a radioisotope.

20. Process for assaying a protein according to any one of Claims 1 to 6, which comprises using monoclonal antibodies which are directed against a protein according to Claim 1 or Claim 2 or an immunogenic sequence of such a protein.

21. Expression vector which comprises a DNA sequence according to any one of Claims 7 to 12.

22. Host which is transformed with a vector according to Claim 21.

23. DNA sequence which essentially comprises, as the promoter sequence, all or part of the sequence represented by the sequence SEQ ID No. 4.

24. Expression vector which comprises, as the promoter sequence, a sequence according to Claim 23.

25. Host which is transformed with a vector according to Claim 24.

26. Process for preparing a protein, a protein fragment or a peptide according to any one of Claims 1 to 6, which comprises inserting a DNA sequence according to any one of Claims 7 to 12 into an expression vector, transforming cells with this expression vector and culturing these cells.

27. Process according to Claim 26, in which the host cells are eukaryotic cells.

28. Process according to Claim 26, in which the host cells are prokaryotic cells.

29. Monoclonal antibodies according to Claim 13 or proteins according to any one of Claims 1 to 6, which are intended for being used as a medicament.

30. Use of monoclonal antibodies according to Claim 13 or of proteins or peptides according to one of Claims 1 to 6 for preparing a medicament which is intended for treating human ailments which are linked to infection with the HIV-type viruses.

31. Use of monoclonal antibodies according to Claim 13 or of proteins or peptides according to any one of Claims 1 to 6 for preparing a medicament which is intended for treating diseases which involve an intercellular adhesion interaction between a ligand and the protein according to Claim 2.

32. Use of monoclonal antibodies according to Claim 13 or of proteins or peptides according to any one of Claims 1 to 6 for preparing a medicament which is intended for treating the human diseases which are caused by viruses which bind specifically to the protein according to Claim 2.

33. Pharmaceutical composition which comprises, as the active principle, a protein or a peptide according to any one of Claims 1 to 6.

## Patentansprüche

1. Proteine, die durch die Peptidsequenz, die durch die Sequenz SEQ-ID-Nr. 1 wiedergegeben ist, oder seine Fraktionen und die Sequenzen gebildet sind, die sich durch eine oder mehrere Aminosäuren unterschieden und die sich mit spezifischen monoklonalen Antikörpern eines Proteins verbinden, das durch die Sequenz SEQ-ID-Nr.1 wiedergegeben ist.

2. Protein nach Anspruch 1, das durch die Peptidsequenz, die durch die SEQ-ID-Nr. 2 wiedergegeben ist, in der Xaa unter H und einem Fragment der Sequenz -28 bis -1 der Proteinfrequenz ausgewählt ist, die durch die Sequenz SEQ-ID-Nr. 1 wiedergegeben ist, und die Sequenzen gebildet ist, die sich durch eine oder mehrere Aminosäuren unterscheiden und die sich mit spezifischen monoklonalen Antikörpern eines Proteins verbinden, das durch die Sequenz SEQ-ID-Nr. 1 wiedergegeben ist.

3. Lösliche Fraktion eines Proteins nach Anspruch 1 oder Anspruch 2, die wenigstens eine der vier extrazellulären Domänen vom Immunoglobulintyp von besagtem Protein umfaßt.

4. Lösliche Fraktion eines Proteins nach Anspruch 1, die den intracytoplasmischen Bereich des besagten Proteins umfaßt.

5. Immunogene Peptidsequenz, die die Sequenz umfaßt, die durch die Sequenz SEQ-ID-Nr. 3 des Proteins nach Anspruch 1 oder Anspruch 2 wiedergegeben ist.

6. Proteine oder Peptide nach einem der Ansprüche 1 bis 5, die einen Methionrest zusätzlich zu dem -NH₂-Ende umfassen.

7. DNA-Sequenz, die für ein Protein nach jedem der Ansprüche 1 bis 6 co-diert, ihre Allelvarianten oder Varianten, die aus der Entartung des genetischen Codes oder aus Punktmutationen resultieren und die für eine Protein nach einem der Ansprüche 1 bis 6 codieren.

8. DNA-Sequenz nach Anspruch 7, die vor allem eine Sequenz umfaßt, die der DNA-Sequenz entspricht, die durch die Sequenz SEQ-ID-Nr. 1 wiedergegeben ist.

9. DNA-Sequenz nach Anspruch 7 oder Anspruch 8, die eine cDNA-Sequenz ist.

10. DNA-Sequenz nach Anspruch 7, die eine genomische DNA-Sequenz ist und die ungefähr 6,6 kb umfaßt und aus 8 Exons besteht, deren erste Nukleotide jeweils die in Position 1, 289, 437, 742, 1012, 1288, 1531 und 1662 der DNA-Sequenz nach Anspruch 8 sind.

11. DNA-Sequenz nach Anspruch 10, dadurch gekennzeichnet, daß ihre Restriktionskarte durch die Endonukleasen Sp I, Xba I, Pst I, Sac I, Bam HI, Hind III, Eco RI so wie in Fig. 2 wiedergegeben ist.

12. DNA-Sequenz, die fähig ist, sich unter den Stringenzbedingungen zu einer DNA-Sequenz nach einem der Ansprüche 7 bis 11 zu hybridisieren.

13. Monoklonale Antikörper, die gegen ein Protein nach Anspruch 1 oder Anspruch 2 oder eine immunogene Sequenz eines derartigen Proteins gerichtet sind.

14. Monoklonale Antikörper nach Anspruch 13, die gegen die Sequenz nach Anspruch 5 gerichtet sind.

15. Fragmente Fab, Fab', F(ab')₂ oder Fv von monoklonalen Antikörpern nach Anspruch 13.

16. Derivate von monoklonalen Antikörpern, die monoklonale Antikörper nach Anspruch 13 oder Anspruch 14 oder Fragmente nach Anspruch 15 umfassen, an die Marker oder therapeutisch aktive Moleküle gebunden sind.

17. Hybridome, die monoklonale Antikörper nach Anspruch 13 erzeugen.

18. Therapeutische Zusammensetzung, die monoklonale Antikörper nach Anspruch 13, Fragmente von monoklonalen Antikörpern nach Anspruch 15 oder Derivate von monoklonalen Antikörpern nach Anspruch 16 umfaßt.

19. Therapeutische Zusammensetzung nach Anspruch 18, in der die Derivate von monoklonalen Antikörpern monoklonale Antikörper oder Fragmente dieser Antikörper sind, die an ein zytotoxisches Molekül oder ein Radioisotop binden.

20. Verfahren der Dosierung eines Proteins nach einem der Ansprüche 1 bis 6, das die Verwendung von monoklonalen Antikörpern umfaßt, die gegen ein Protein nach Anspruch 1 oder Anspruch 2 oder eine immumogene Sequenz eines derartigen Proteins gerichtet sind.

21. Expressionsvektor, der eine DNA-Sequenz nach einem der Ansprüche 7 bis 12 umfaßt.

22. Transformierter Wirt mit einem Vektor nach Anspruch 21.

23. DNA-Sequenz, die vor allem als Initiatorsequenz ganz oder teilweise die Sequenz umfaßt, die durch die Sequenz SEQ-ID-Nr. 4 wiedergegeben ist.

24. Expressionsvektor, der als Initiatorsequenz eine Sequenz nach Anspruch 23 umfaßt.

25. Transformierter Wirt mit einem Vektor nach Anspruch 24.

26. Verfahren der Herstellung eines Proteins, eines Proteinfragmentes oder eines Peptids nach einem der Ansprüche 1 bis 6, das die Insertion einer DNA-Sequenz nach einem der Ansprüche 7 bis 12 in einen Expressionsvektor, die Zelltransformation mit diesem Expressionsvektor und die Züchtung dieser Zellen umfaßt.

27. Verfahren nach Anspruch 26, in dem die Wirtszellen eukaryotische Zellen sind.

28. Verfahren nach Anspruch 26, in dem die Wirtszellen prokaryotische Zellen sind.

29. Monoklonale Antikörper nach Anspruch 13 oder Proteine nach einem der Ansprüche 1 bis 6, die zur Verwendung als Medikament bestimmt sind.

30. Verwendung von monoklonalen Antikörpern nach Anspruch 13 oder von Proteinen oder Peptiden nach einem der Ansprüche 1 bis 6 zur Herstellung eines Medikamentes, das zur Behandlung von menschlichen Erkrankungen bestimmt ist, die mit einer Infektion durch die Viren des HIV-Typs verbunden sind.

31. Verwendung von monoklonalen Antikörpern nach Anspruch 13 oder von Proteinen oder Peptiden nach einem der Ansprüche 1 bis 6 zur Herstellung eines Medikamentes, das zur Behandlung von Leiden bestimmt ist, die eine Interaktion von interzellulärer Adhäsion zwischen einem Liganden und dem Protein nach Anspruch 2 eingreifen lassen.

32. Verwendung von monoklonalen Antikörpern nach Anspruch 13 oder von Proteinen oder Peptiden nach einem der Ansprüche 1 bis 6 zur Herstellung eines Medikamentes, das zur Behandlung von menschlichen Leiden bestimmt ist, die durch Viren verursacht werden, die sich auf spezifische Art mit dem Protein nach Anspruch 2 verbinden.

33. Pharmazeutische Zusammensetzung, die als aktiven Hauptgehalt ein Protein oder ein Peptid nach einem der Ansprüche 1 bis 6 umfaßt.
